# EUROPEAN PATENT APPLICATION

(11) **EP 4 477 746 A1**
(43) Date of publication of application: **18.12.2024**
(21) Application number: 23752928.4
(22) Date of filing: 09.02.2023
(51) Int. Cl.: C12N 5/0775, C12N 5/0793

(54) **METHOD FOR ASSESSING DIFFERENTIATION POTENTIAL OF CELLS IN CULTURE BROTH IN DIFFERENTIATION OF PLURIPOTENT STEM CELLS INTO NEURAL CELLS OF MIDBRAIN FLOOR PLATE REGION**

(30) Priority: 09.02.2022 JP 2022018595
(71) Applicant: Sumitomo Pharma Co., Ltd., Osaka-shi, Osaka 541-0045 (JP); Kyoto University, Kyoto-shi, Kyoto 606-8501 (JP)
(72) Inventor: NAKANE, Atsushi, Kobe-shi, Hyogo 650-0047 (JP); YOSHIDA, Kenji, Kobe-shi, Hyogo 650-0047 (JP); AOYAGI, Yuka, Kobe-shi, Hyogo 650-0047 (JP); IKEDA, Megumi, Kobe-shi, Hyogo 650-0047 (JP); TAKAHASHI, Jun, Kyoto-shi, Kyoto 606-8501 (JP); DOI, Daisuke, Kyoto-shi, Kyoto 606-8501 (JP)
(74) Representative: J A Kemp LLP
(86) International application number: PCT/JP2023/004282
(87) International publication number: WO 2023/153464

(57) **Abstract**

A method for assessing differentiation potential of cells in a culture broth in the differentiation of pluripotent stem cells into neural cells of the midbrain floor plate region, comprising: measuring a concentration of NT-3 in a culture supernatant in a culture broth obtained by culturing the pluripotent stem cells in a medium containing an inducer of differentiation into the neural cells of the midbrain floor plate region; comparing the measured concentration of NT-3 with a reference concentration; and when the concentration of NT-3 is equal to or more than the reference concentration, assessing the cells in the culture broth as capable of differentiating into the neural cells of the midbrain floor plate region, wherein the culture supernatant is a culture supernatant collected from the culture broth at any time from 48 hours to 240 hours after the start of culture of the pluripotent stem cells.

## Description

### TECHNICAL FIELD

The present invention relates to a method for assessing differentiation potential of cells in a culture broth in the differentiation of pluripotent stem cells into neural cells of the midbrain floor plate region.

### BACKGROUND ART

Parkinson's disease is a neurodegenerative disease that is caused by a loss of dopaminergic neurons in the midbrain substantia nigra. Hence, a method of artificially manufacturing the dopaminergic neurons or dopaminergic progenitor cells by the induction of differentiation of pluripotent stem cells, and transplanting them into the brain of a patient is expected as an effective treatment method for Parkinson's disease, and a method for manufacturing a cell medicament therefrom is under study.

Meanwhile, neurotrophin-3 (NT-3) is a member of the nerve growth factor (NGF) family and is known to promote the survival and differentiation of neuronal cells in the central nervous system and synapses. NT-3 is used in the step of maturing neural cells of the midbrain floor plate region, which correspond to intermediates, into dopaminergic progenitor cells in manufacturing dopaminergic progenitor cell from pluripotent stem cells (PTL 1). As for living bodies, for example, NPL 1 has reported that the NT-3 gene is expressed in neural cells. However, it has not been known so far that NT-3 is involved in the induction of differentiation at an initial stage of manufacturing dopaminergic progenitor cells through neural progenitor cells and neural cells of the midbrain floor plate region from pluripotent stem cells. It has not been known either that NT-3 is secreted into a culture supernatant in the process of the differentiation of neural cells of the midbrain floor plate region.

### CITATION LIST

### PATENT LITERATURE

PTL 1: U.S. Patent No. 7250294

### NON PATENT LITERATURE

NPL 1: Bernd P., Gene Expr. 2008; 14 (4): 241-50
NPL 2: Frontiers in Cell and Developmental Biology, August 2020, Volume 8, Article 729

### SUMMARY OF INVENTION

### TECHNICAL PROBLEM

A plurality of research groups have studied a method for manufacturing dopaminergic progenitor cells by inducing the differentiation of pluripotent stem cells. However, a feature of a method for inducing the differentiation of pluripotent stem cells into neural cells such as dopaminergic progenitor cells is complicated steps which require a long period for obtaining cells of interest. Hence, there is a demand for a method for predicting that the induction of differentiation progresses smoothly and that a normal lot containing more than a given ratio of cells of interest suitable for transplantation to humans can be provided, at an early stage of induction of differentiation in a method for manufacturing dopaminergic progenitor cells.

Also, an index for noninvasively monitoring the state of cells in the process of the induction of differentiation is required for confirming over time that a differentiation step progresses normally.

The present invention has been made in light of the situation described above. An object of the present invention is to provide a method for assessing differentiation potential into neural cells of the midbrain floor plate region, which correspond to intermediates, in the process of the differentiation of pluripotent stem cells into dopaminergic progenitor cells, the method being capable of making assessment at an early stage of induction of differentiation of pluripotent stem cells into dopaminergic progenitor cells.

### SOLUTION TO PROBLEM

The present inventors have pursued diligent studies to attain the object and consequently completed the present invention by finding that differentiation potential of pluripotent stem cells into neural cells of the midbrain floor plate region can be assessed at an early stage of induction of differentiation by monitoring the concentration of neurotrophin-3 (hereinafter, also referred to as "NT-3") secreted into a culture supernatant in the process of the differentiation of the pluripotent stem cells into dopaminergic progenitor cells. Specifically, the present invention is as follows.
[1] A first method for assessing differentiation potential of cells in a culture broth in the differentiation of pluripotent stem cells into neural cells of the midbrain floor plate region according to the present invention is
   a method for assessing differentiation potential of cells in a culture broth in the differentiation of pluripotent stem cells into neural cells of the midbrain floor plate region, comprising:
   measuring a concentration of NT-3 in a culture supernatant in a culture broth obtained by culturing the pluripotent stem cells in a medium containing an inducer of differentiation into the neural cells of the midbrain floor plate region;
   comparing the measured concentration of NT-3 with a reference concentration; and
   when the concentration of NT-3 is equal to or more than the reference concentration, assessing the cells in the culture broth as capable of differentiating into the neural cells of the midbrain floor plate region, wherein
   the culture supernatant is a culture supernatant collected from the culture broth at any time from 48 hours to 240 hours (or 48 hours to 96 hours) after the start of culture of the pluripotent stem cells.
[2] A second method for assessing differentiation potential of cells in a culture broth in the differentiation of pluripotent stem cells into neural cells of the midbrain floor plate region according to the present invention is
   a method for assessing differentiation potential of cells in a culture broth in the differentiation of pluripotent stem cells into neural cells of the midbrain floor plate region, comprising:
   measuring a concentration of NT-3 in each of a first culture supernatant and a second culture supernatant in a culture broth obtained by culturing the pluripotent stem cells in a medium containing an inducer of differentiation into the neural cells of the midbrain floor plate region;
   determining a rate of change in the concentration of NT-3 from the concentration of NT-3 in the first culture supernatant and the concentration of NT-3 in the second culture supernatant;
   comparing an absolute value of the rate of change in the concentration of NT-3 with a reference rate of change; and
   when the absolute value of the rate of change in the concentration of NT-3 is equal to or more than the reference rate of change, assessing the cells in the culture broth as capable of differentiating into the neural cells of the midbrain floor plate region, wherein
   the first culture supernatant is a culture supernatant collected from the culture broth at any time from 48 hours to 192 hours (or 48 hours to 96 hours) after the start of culture of the pluripotent stem cells, and
   the second culture supernatant is a culture supernatant collected from the culture broth at any time from 24 hours to 96 hours (or 24 hours to 72 hours) before collection of the first culture supernatant or at any time from 24 hours to 96 hours (or 24 hours to 72 hours) after collection of the first culture supernatant.
[3] A third method for assessing differentiation potential of cells in a culture broth in the differentiation of pluripotent stem cells into neural cells of the midbrain floor plate region according to the present invention is
   a method for assessing differentiation potential of cells in a culture broth in the differentiation of pluripotent stem cells into neural cells of the midbrain floor plate region, comprising:
   measuring a concentration of NT-3 in each of a first culture supernatant, a second culture supernatant, and a third culture supernatant in a culture broth obtained by culturing the pluripotent stem cells in a medium containing an inducer of differentiation into the neural cells of the midbrain floor plate region;
   comparing the concentration of NT-3 in the first culture supernatant, the concentration of NT-3 in the second culture supernatant, and the concentration of NT-3 in the third culture supernatant; and
   when the concentration of NT-3 in the first culture supernatant is higher than the concentration of NT-3 in the second culture supernatant and the concentration of NT-3 in the third culture supernatant, assessing the cells in the culture broth as capable of differentiating into the neural cells of the midbrain floor plate region, wherein
   the first culture supernatant is a culture supernatant collected from the culture broth at any time from 48 hours to 192 hours (or 48 hours to 96 hours) after the start of culture of the pluripotent stem cells,
   the second culture supernatant is a culture supernatant collected from the culture broth at any time from 24 hours to 96 hours (or 24 hours to 72 hours) before collection of the first culture supernatant, and
   the third culture supernatant is a culture supernatant collected from the culture broth at any time from 24 hours to 96 hours (or 24 hours to 72 hours) after collection of the first culture supernatant.
[4] According to any of [1] to [3], preferably, the inducer of differentiation into the neural cells of the midbrain floor plate region comprises at least one SMAD signaling-inhibiting substance.
[5] According to [4], preferably, the SMAD signaling-inhibiting substance comprises at least one BMP signaling-inhibiting substance and at least one TGFβ signaling-inhibiting substance.
[6] According to [5], preferably,
   the BMP signaling-inhibiting substance comprises at least one member selected from the group consisting of LDN-193189, Noggin, DMH1, Chordin, Follistatin, K02288, LDN-214117, LDN-212854, ML347 (LDN193719), and Dorsomorphin, and
   the TGFβ signaling-inhibiting substance comprises at least one member selected from the group consisting of SB-431542, A-83-01, Lefty-1, Lefty-2, Galunisertib (LY2157299), SB-202190, SB-525334, SB-505124, NPC30345, Prifenidone (S-7701), GW788388, E-616452 (RepSox), SD208, TP0427736, BIBF-0775, LY3200882, Vactosertib (TEW-7197), ITD-1, SD093, SD908, LY2109761, LY364947, and LY580276.
   More preferably, the TGFβ signaling-inhibiting substance comprises at least one member selected from the group consisting of SB-431542, A-83-01, Lefty-1, Lefty-2, Galunisertib (LY2157299), SB-202190, SB-525334, SB-505124, NPC30345, Prifenidone (S-7701), GW788388, E-616452 (RepSox), SD208, ITD-1, SD093, SD908, LY2109761, LY364947, and LY580276.
[7] According to any of [1] to [6], preferably, the inducer of differentiation into the neural cells of the midbrain floor plate region further comprises a SHH signaling-activating substance and/or a Wnt signaling-activating substance.
[8] According to any of [1] to [6], preferably, the medium further contains a SHH signaling-activating substance and a Wnt signaling-activating substance.
[9] According to [7] or [8], preferably, the SHH signaling-activating substance comprises at least one member selected from the group consisting of SHH and a fragment (e.g., Shh (C24II) N-Terminus and Shh (C25II) N-Terminus) and modified form thereof, a SHH receptor, a SHH receptor agonist, Hh-Ag1.5, Smoothened Agonist, 20a-hydroxycholesterol, Purmorphamine, and SAG.
[10] According to [7], [8], or [9], preferably, the Wnt signaling-activating substance comprises at least one member selected from the group consisting of WNT3A, and a GSK-3β-inhibiting substance.
[11] According to [10], preferably, the GSK-3β-inhibiting substance comprises at least one member selected from the group consisting of CHIR99021, BIO, CHIR98014, SKL2001, SB216763, GSK-3β inhibitor VII (4-dibromoacetophenone), and L803-mts.
[12] According to any of [1] to [11], preferably, the pluripotent stem cells are iPS cells or ES cells.
[13] A method for manufacturing dopaminergic progenitor cells or precursor cells thereof according to the present invention is
   a method for manufacturing dopaminergic progenitor cells or precursor cells thereof from pluripotent stem cells, comprising:
   (A) starting the culture of the pluripotent stem cells under culture conditions capable of inducing differentiation into neural cells of the midbrain floor plate region;
   (B) performing a method for assessing differentiation potential of cells in a culture broth in the differentiation of pluripotent stem cells into neural cells of the midbrain floor plate region according to any of [1] to [12] between 0 hours and 288 hours after the start of culture of the pluripotent stem cells;
   (C) determining to further continue the culture of the cells in the culture broth assessed as capable of differentiating into the neural cells of the midbrain floor plate region in the step (B), and allowing the cells in the culture broth to differentiate into the neural cells of the midbrain floor plate region; and
   (D) culturing the neural cells of the midbrain floor plate region under culture conditions capable of inducing differentiation into the dopaminergic progenitor cells so that the cells are allowed to differentiate into the dopaminergic progenitor cells or precursor cells thereof.
[14] According to [13], preferably, the method for manufacturing dopaminergic progenitor cells or precursor cells thereof further comprises, between the step (C) and the step (D),
   (E) collecting the neural cells of the midbrain floor plate region obtained in the step (C).
[15] According to [13] or [14], preferably, the step (D) of the method for manufacturing dopaminergic progenitor cells or precursor cells thereof is performed by selecting and collecting midbrain floor plate cells from the neural cells of the midbrain floor plate region, and culturing the collected midbrain floor plate cells under culture conditions capable of inducing differentiation into the dopaminergic progenitor cells. Specifically, properly differentiated midbrain floor plate cells of interest can be selected and collected from a cell population of the neural cells of the midbrain floor plate region (cell population comprising midbrain floor plate cells) and then induced to differentiate into dopaminergic progenitor cells or precursor cells thereof by suspension culture or adhesion culture.

### ADVANTAGEOUS EFFECTS OF INVENTION

The present invention can provide a method for assessing differentiation potential of pluripotent stem cells into neural cells of the midbrain floor plate region, the method being capable of assessing the differentiation potential at an early stage of induction of differentiation. According to the present invention, the differentiation potential can be assessed in a manner noninvasive to cells at an early stage of induction of differentiation. Hence, cost ascribable to the continuation of a differentiation culture lot inferior in differentiation potential can also be reduced.

### BRIEF DESCRIPTION OF DRAWINGS

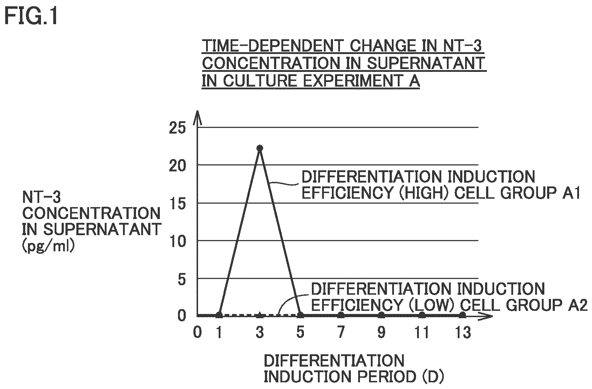

Fig. 1 is a graph showing time-dependent change in NT-3 concentration in a culture supernatant as to cell groups A1 and A2 differing in differentiation efficiency in culture experiment A. The abscissa depicts the number of culture days for the induction of differentiation, and the ordinate depicts the NT-3 concentration in the supernatant. This data shows that the NT-3 concentration is elevated under a condition of high differentiation induction efficiency.

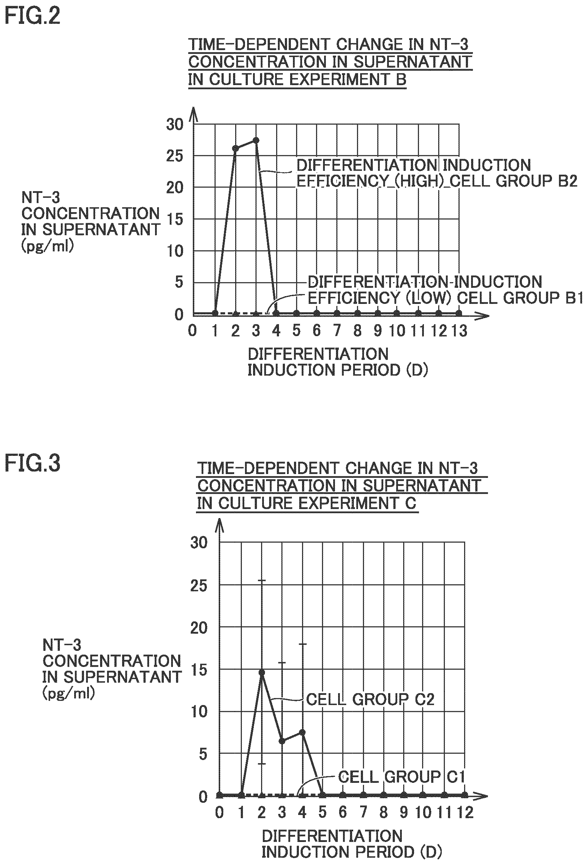

Fig. 2 is a graph showing time-dependent change in NT-3 concentration in a culture supernatant as to cell groups B1 and B2 differing in differentiation efficiency in culture experiment B. The abscissa depicts the number of culture days for the induction of differentiation, and the ordinate depicts the NT-3 concentration in the supernatant. This data shows that the NT-3 concentration is elevated under a condition of high differentiation induction efficiency.
Fig. 3 is a graph showing time-dependent change in NT-3 concentration in a culture supernatant as to cell groups C1 and C2 differing in differentiation efficiency in culture experiment C. The abscissa depicts the number of culture days for the induction of differentiation, and the ordinate depicts the NT-3 concentration in the supernatant. This data shows that the NT-3 concentration is elevated under a condition of high differentiation induction efficiency.

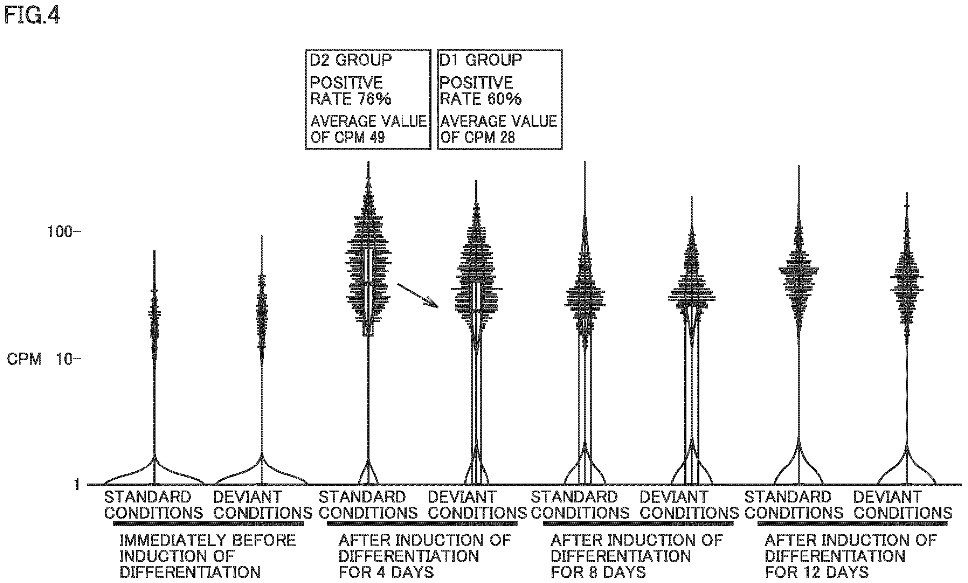

Fig. 4 is a graph showing, in a violin plot, results of analyzing single-cell gene expression as to cell groups D1 and D2 differing in differentiation efficiency in culture experiment D. The abscissa depicts the number of culture days for the induction of differentiation and culture conditions, and the ordinate depicts Count per Million (CPM) serving as an index for an average RNA expression level. The data in this graph shows that many cells highly express NT-3 gene (NTF3) under a condition of high differentiation induction efficiency.

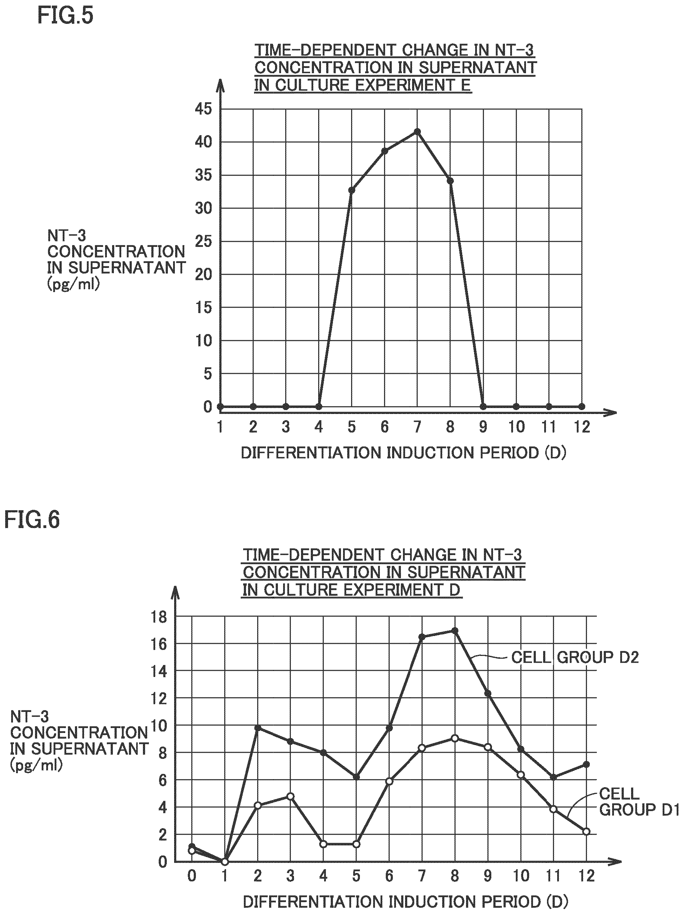

Fig. 5 is a graph showing time-dependent change in NT-3 concentration in a culture supernatant in differentiation culture using iPS cell line established using a Sendai virus vector in culture experiment E. The abscissa depicts the number of culture days for the induction of differentiation, and the ordinate depicts the NT-3 concentration in the supernatant. This data shows that the NT-3 concentration is elevated as differentiation progresses.
Fig. 6 is a graph showing time-dependent change in NT-3 concentration in a culture supernatant as to cell groups D1 and D2 differing in differentiation efficiency in culture experiment D. The abscissa depicts the number of culture days for the induction of differentiation, and the ordinate depicts the NT-3 concentration in the supernatant. This data shows that the NT-3 concentration is elevated.

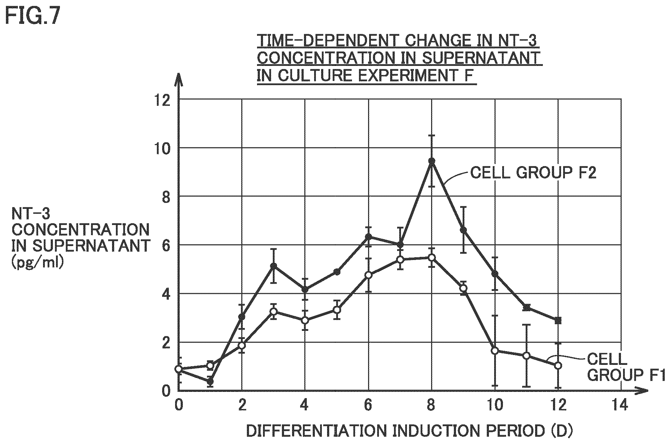

Fig. 7 is a graph showing time-dependent change in NT-3 concentration in a culture supernatant as to cell groups F1 and F2 differing in culture conditions in culture experiment F. The abscissa depicts the number of culture days for the induction of differentiation, and the ordinate depicts the NT-3 concentration in the supernatant. This data shows that the NT-3 concentration is elevated.

### DESCRIPTION OF EMBODIMENTS

Hereinafter, one embodiment of the present invention (hereinafter, also referred to as the "present embodiment") will be described. However, the present embodiment is not limited by those described herein. In the present specification, the term "A to Z" means the upper and lower limits of a range (i.e., A or more and Z or less). When the unit is not described for A and is described only for Z, the unit of A is the same as that of Z.

### <<Method for assessing differentiation potential of cells in culture broth in differentiation of pluripotent stem cells into neural cells of midbrain floor plate region - (1)>>

The first method for assessing differentiation potential of cells in a culture broth in the differentiation of pluripotent stem cells into neural cells of the midbrain floor plate region according to the present embodiment is
a method for assessing differentiation potential of cells in a culture broth in the differentiation of pluripotent stem cells into neural cells of the midbrain floor plate region, comprising:
measuring a concentration of NT-3 in a culture supernatant in a culture broth obtained by culturing the pluripotent stem cells in a medium containing an inducer of differentiation into the neural cells of the midbrain floor plate region;
comparing the measured concentration of NT-3 with a reference concentration; and
when the concentration of NT-3 is equal to or more than the reference concentration, assessing the cells in the culture broth as capable of differentiating into the neural cells of the midbrain floor plate region, wherein
the culture supernatant is a culture supernatant collected from the culture broth at any time from 48 hours to 240 hours after the start of culture of the pluripotent stem cells. Hereinafter, detailed description will be made.

### <Pluripotent stem cells>

In the present embodiment, the "pluripotent stem cells" mean stem cells that have pluripotency allowing for differentiation into every cell present in a living body, i.e., three germ layers endoderm, mesoderm, and ectoderm, and also have proliferative capacity. Examples of the pluripotent stem cells include, but are not particularly limited to, embryonic stem (ES) cells, embryonic stem cells derived from a cloned embryo obtained by nuclear transfer (ntES) cells, germline stem cells (GS cells), embryonic germ cells (EG cells), induced pluripotent stem (iPS) cells, and pluripotent cells derived from cultured fibroblasts or bone marrow stem cells (Muse cells). The pluripotent stem cells are preferably at least one member selected from the group consisting of ES cells, ntES cells, and iPS cells. In one aspect of the present embodiment, the pluripotent stem cells are preferably iPS cells or ES cells. An organism from which the pluripotent stem cells are derived is not particularly limited. Mammal-derived pluripotent stem cells are preferred, and primate-derived pluripotent stem cells are more preferred. Specific examples thereof include mouse-, human-, or monkey-derived pluripotent stem cells.

### (A) Embryonic stem cells

The ES cells are stem cells that are established from a mammalian (e.g., human or mouse) early embryo (e.g., blastocyst), specifically, an inner cell mass within 14 days after fertilization, and have pluripotency and proliferative capacity through self-renewal. The ES cells are embryo-derived stem cells derived from an inner cell mass of blastocyst, which is an embryo after the eight-cell stage and morula of a fertilized egg. The ES cells have the ability to differentiate into every cell constituting an adult body, i.e., pluripotent differentiation, and proliferative capacity through self-renewal. The ES cells were discovered in mice in 1981 (M.J. Evans and M.H. Kaufman (1981), Nature 292: 154-156). Then, ES cell lines were established for primates such as humans or monkeys (J.A. Thomson et al. (1998), Science 282: 1145-1147; J.A. Thomson et al. (1995), Proc. Natl. Acad. Sci. USA, 92: 7844-7848; J.A. Thomson et al. (1996), Biol. Reprod., 55: 254-259; and J.A. Thomson and V.S. Marshall (1998), Curr. Top. Dev. Biol., 38: 133-165).

The ES cells can be established by isolating an inner cell mass from the blastocyst of a fertilized egg of a subject animal, and culturing the inner cell mass on feeder fibroblasts. The cells can be maintained by subculture using a culture broth supplemented with a substance such as leukemia inhibitory factor (LIF) or basic fibroblast growth factor (bFGF). Methods for establishing and maintaining human and monkey ES cells are described in, for example, USP5,843,780; Thomson JA, et al. (1995), Proc. Natl. Acad. Sci. USA. 92: 7844-7848; Thomson JA, et al. (1998), Science. 282: 1145-1147; H. Suemori et al. (2006), Biochem. Biophys. Res. Commun., 345: 926-932; M. Ueno et al. (2006), Proc. Natl. Acad. Sci. USA, 103: 9554-9559; H. Suemori et al. (2001), Dev. Dyn., 222: 273-279; H. Kawasaki et al. (2002), Proc. Natl. Acad. Sci. USA, 99: 1580-1585; and Klimanskaya I, et al. (2006), Nature. 444: 481-485.

For example, DMEM/F-12 medium replenished with 0.1 mM 2-mercaptoethanol, 0.1 mM nonessential amino acid, 2 mM L-glutamic acid, 20% KSR (KnockOut serum replacement), and 4 ng/ml bFGF is used as a medium for ES cell preparation, and human ES cells can be maintained at 37°C in a wet atmosphere of 2% CO₂/98% air (O. Fumitaka et al. (2008), Nat. Biotechnol., 26: 215-224). The ES cells need to be passaged every 3 to 4 days. This passage can be performed using, for example, 0.25% trypsin and 0.1 mg/ml collagenase IV in PBS containing 1 mM CaCl₂ and 20% KSR.

The ES cells can generally be selected by Real-Time PCR with the expression of a gene marker such as alkaline phosphatase, Oct-3/4, or Nanog as an index. Particularly, human ES cells can be selected with the expression of a gene marker such as OCT-3/4, NANOG, or ECAD as an index (E. Kroon et al. (2008), Nat. Biotechnol., 26: 443-452). A human ES cell line is available from a predetermined facility. For example, WA01 (H1) and WA09 (H9) are available from WiCell Research Institute. KhES-1, KhES-2, and KhES-3 are available from Institute for Life and Medical Sciences, Kyoto University (Kyoto, Japan).

### (B) Germline stem cells

The germline stem cells are pluripotent stem cells derived from the testis and are cells of origin for spermatogenesis. The germline stem cells, as in ES cells, can be induced to differentiate into cells of various lineages and have properties, for example, by which chimeric mice can be produced when the cells are transplanted to mouse blastocyst (M. Kanatsu-Shinohara et al. (2003) Biol. Reprod., 69: 612-616; and K. Shinohara et al. (2004), Cell, 119: 1001-1012). The germline stem cells are capable of self-renewing in a medium containing glial cell line-derived neurotrophic factor (GDNF). The germline stem cells can be obtained by repeating a passage under culture conditions similar to those for ES cells (Masanori Takebayashi, et al. (2008), Experimental Medicine, Vol. 26, No. 5 (extra edition), p. 41-46, Yodosha Co., Ltd. (Tokyo, Japan)).

### (C) Embryonic germ cells

The embryonic germ cells are cells that are established from primordial germ cells during fetal life and have pluripotency similar to that of ES cells. The embryonic germ cells can be established by culturing primordial germ cells in the presence of a substance such as LIF, bFGF, or stem cell factor (Y. Matsui et al. (1992), Cell, 70: 841-847; and J.L. Resnick et al. (1992), Nature, 359: 550-551).

### (D) Induced pluripotent stem cells

The induced pluripotent stem cells (iPS cells) are somatic cell-derived artificial stem cells that can be prepared by introducing a particular reprogramming factor in the form of DNA or a protein to somatic cells, and have characteristics almost equivalent to ES cells, for example, pluripotent differentiation and proliferative capacity through self-renewal (K. Takahashi and S. Yamanaka (2006) Cell, 126: 663-676; K. Takahashi et al. (2007), Cell, 131: 861-872; J. Yu et al. (2007), Science, 318: 1917-1920; Nakagawa, M. et al., Nat. Biotechnol. 26: 101-106 (2008); and WO2007/069666).

The reprogramming factor may be constituted by a gene specifically expressed in ES cells, a gene product or non-cording RNA thereof, or a gene that plays an important role in maintaining the undifferentiated state of ES cells, a gene product or non-cording RNA thereof, or a low-molecular compound. Examples of the gene included in the reprogramming factor include Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tcl1, beta-catenin, Lin28b, Sall1, Sall4, Esrrb, Nr5a2, Tbx3, and Glis1. These reprogramming factors may each be used singly or may be used in combination.

Examples of the combination of the reprogramming factors include combinations described in WO2007/069666, WO2008/118820, WO2009/007852, WO2009/032194, WO2009/058413, WO2009/057831, WO2009/075119, WO2009/079007, WO2009/091659, WO2009/101084, WO2009/101407, WO2009/102983, WO2009/114949, WO2009/117439, WO2009/126250, WO2009/126251, WO2009/126655, WO2009/157593, WO2010/009015, WO2010/033906, WO2010/033920, WO2010/042800, WO2010/050626, WO2010/056831, WO2010/068955, WO2010/098419, WO2010/102267, WO2010/111409, WO2010/111422, WO2010/115050, WO2010/124290, WO2010/147395, WO2010/147612, Huangfu D, et al. (2008), Nat. Biotechnol., 26: 795-797, Shi Y, et al. (2008), Cell Stem Cell, 2: 525-528, Eminli S, et al. (2008), Stem Cells. 26: 2467-2474, Huangfu D, et al. (2008), Nat Biotechnol. 26: 1269-1275, Shi Y, et al. (2008), Cell Stem Cell, 3, 568-574, Zhao Y, et al. (2008), Cell Stem Cell, 3: 475-479, Marson A, (2008), Cell Stem Cell, 3, 132-135, Feng B, et al. (2009), Nat Cell Biol. 11: 197-203, R.L. Judson et al., (2009), Nat. Biotech., 27: 459-461, Lyssiotis CA, et al. (2009), Proc Natl Acad Sci USA106: 8912-8917, Kim JB, et al. (2009), Nature. 461: 649-643, Ichida JK, et al. (2009), Cell Stem Cell. 5: 491-503, Heng JC, et al. (2010), Cell Stem Cell. 6: 167-74, Han J, et al. (2010), Nature. 463: 1096-100, Mali P, et al. (2010), Stem Cells. 28: 713-720, and Maekawa M, et al. (2011), Nature. 474: 225-9.

Preferred examples of the combination of the reprogramming factors can specifically include (1) Oct3/4, Sox2, Klf4, and Myc (c-Myc or L-Myc), (2) Oct3/4, Sox2, Klf4, Lin28, and L-Myc (Stem Cells, 2013; 31: 458-466), and (3) Oct3/4, Sox2, Nanog, and Lin28 (Science, 318, 1917-1920).

In addition to the method for manufacturing the induced pluripotent stem cells by direct reprogramming through gene expression, the induced pluripotent stem cells can also be induced from somatic cells by the addition of a compound or the like (Science, 2013, 341, pp. 651-654).

The reprogramming factor also includes a factor that is used for the purpose of enhancing establishment efficiency, such as a histone deacetylase (HDAC) inhibitor [e.g., low-molecular inhibitors such as valproic acid (VPA), trichostatin A, sodium butyrate, MC1293, and M344, and nucleic acid-derived expression inhibitors such as siRNA and shRNA against HDAC (e.g., HDAC1 siRNA Smartpool (Millipore) and HuSH 29mer shRNA Constructs against HDAC1 (OriGene))], a MEK inhibitor (e.g., PD184352, PD98059, U0126, SL327, and PD0325901), a glycogen synthase kinase-3 inhibitor (e.g., Bio and CHIR99021), a DNA methyltransferase inhibitor (e.g., 5-azacytidine), a histone methyltransferase inhibitor (e.g., low-molecular inhibitors such as BIX-01294, and nucleic acid-derived expression inhibitors such as siRNA and shRNA against Suv39hl, Suv39h2, SetDBl, and G9a), a L-channel calcium agonist (e.g. Bayk8644), butyric acid, a TGFβ inhibitor or an ALK5 inhibitor (e.g., LY364947, SB431542, 616453, and A-83-01), a p53 inhibitor (e.g., siRNA and shRNA against p53), an ARID3A inhibitor (e.g., siRNA and shRNA against ARID3A), miRNA such as miR-291-3p, miR-294, miR-295, and mir-302, Wnt Signaling (e.g., soluble Wnt3a), neuropeptide Y, prostaglandins (e.g., prostaglandin E2 and prostaglandin J2), hTERT, SV40LT, UTF1, IRX6, GLISl, PITX2, and DMRTBl. In the present specification, the pluripotent stem cells also encompass induced pluripotent stem cells established using the aforementioned factors used for the purpose of improving establishment efficiency thereof.

When the reprogramming factor is in the form of a protein, the reprogramming factor may be introduced into somatic cells by, for example, an approach such as lipofection, fusion with a cell-penetrating peptide (e.g., HIV-derived TAT and polyarginine), or microinjection.

When the reprogramming factor is in the form of DNA, the reprogramming factor can be introduced into somatic cells by, for example, an approach such as a vector (e.g., virus, plasmid, or artificial chromosome), lipofection, or microinjection. Examples of the virus vector include retrovirus vectors and lentivirus vectors (all described in Cell, 126, pp. 663-676, 2006; Cell, 131, pp. 861-872, 2007; and Science, 318, pp. 1917-1920, 2007), adenovirus vectors (Science, 322, 945-949, 2008), adeno-associated virus vectors, and Sendai virus vectors (WO2010/008054). Examples of the artificial chromosome vector include human artificial chromosome (HAC), yeast artificial chromosome (YAC), and bacterial artificial chromosome (BAC and PAC). A plasmid for a mammalian cell may be used as the plasmid (Science, 322: 949-953, 2008). The vector can comprise control sequences such as a promoter, an enhancer, a ribosomal binding sequence, a terminator, and a polyadenylation site so as to enable the nuclear reprogramming substance to be expressed. The vector can optionally further comprise a drug resistance gene (e.g., kanamycin resistance gene, ampicillin resistance gene, and puromycin resistance gene), a selective marker sequence such as thymidine kinase gene or diphtheria toxin gene, or a reporter gene sequence such as green fluorescent protein (GFP), β glucuronidase (GUS), or FLAG. The vector may have a gene encoding the reprogramming factor, or a promoter and the reprogramming factor-encoding gene linked thereto as well as LoxP sequences flanking the gene or the promoter and the gene. The resulting vector, after being introduced to somatic cells, enables the gene encoding reprogramming factor, or the promoter and the reprogramming factor-encoding gene linked thereto together to be cleaved.

When the reprogramming factor is in the form of RNA, the reprogramming factor may be introduced into somatic cells by, for example, an approach such as lipofection or microinjection. RNA in which 5-methylcytidine and pseudouridine (TriLink Biotechnologies) are incorporated may be used for suppressing degradation (Warren L, (2010) Cell Stem Cell. 7: 618-630).

Examples of the medium for the establishment and maintenance culture of the iPS cells include DMEM, DMEM/F12, or DME medium containing 10 to 15% FBS, commercially available media [e.g., a medium for human ES/iPS cell culture (AK03N, Ajinomoto Co., Inc.), a medium for mouse ES cell culture (TX-WES medium, Thrombo-X), and a medium for primate ES cell culture (medium for a primate ES/iPS cell, REPROCELL Inc.), and serum-free media (mTeSR, STEMCELL Technologies, and Essentials, Life technologies corp.)]. The DMEM, DMEM/F12, or DME medium mentioned above can appropriately further contain LIF, penicillin/streptomycin, puromycin, L-glutamine, nonessential amino acids, 2-mercaptoethanol, or the like.

Examples of the culture method include the following methods. First, somatic cells are contacted with the reprogramming factor in DMEM or DMEM/F12 medium containing 10% FBS at 37°C in a 5% CO₂ environment and cultured for approximately 4 to 7 days. Then, the somatic cells are reseeded over feeder cells (e.g., mitomycin C-treated STO cells and SNL cells) and cultured in a medium for primate ES cell culture containing bFGF from approximately 10 days after contact of the somatic cells with the reprogramming factor. Finally, an iPS-like colony can be produced by culture for approximately 30 to approximately 45 days or more from the contact.

Alternatively, the cells are cultured in a DMEM medium containing 10% FBS (this medium can appropriately further contain LIF, penicillin/streptomycin, puromycin, L-glutamine, nonessential amino acids, 2-mercaptoethanol, or the like) over feeder cells (e.g., mitomycin C-treated STO cells and SNL cells) at 37°C in a 5% CO₂ environment so that an iPS-like colony can be produced approximately 25 to approximately 30 days or more.

Desirably, the iPS cells can be manufactured in the absence of feeder cells (feeder-free). Specific examples of such an approach include a method of using somatic cells themselves to be reprogrammed instead of the feeder cells (Takahashi K, et al. (2009), PLoS One. 4: e8067 or WO2010/137746), or extracellular matrix (e.g., Laminin-5 (WO2009/123349) and Matrigel (BD)).

Other examples thereof include a method of performing culture using a medium containing no serum (Sun N, et al. (2009), Proc Natl Acad Sci USA. 106: 15720-15725). Further, the iPS cells may be established under hypoxic conditions (oxygen concentration of 0.1% or more and 15% or less) in order to enhance establishment efficiency (Yoshida Y, et al. (2009), Cell Stem Cell. 5: 237-241 or WO2010/013845).

During the culture, the medium is replaced with a fresh medium once a day from 2 days after the start of culture. The number of somatic cells for use in reprogramming is not limited and is preferably in the range of approximately 5 × 10³ to approximately 5 × 10⁶ cells per 100 cm² of a culture dish.

The iPS cells can be selected from the shape of the formed colony. When a drug resistance gene to be expressed in conjunction with a gene, such as Oct3/4 or Nanog, which is expressed by the reprogramming of the somatic cells, is introduced as a marker gene, the established iPS cells can be selected by culture in a medium containing the corresponding drug (selective medium). When the marker gene is a fluorescent protein gene, the iPS cells can be selected by observation under a fluorescence microscope. When the marker gene is a luminescent enzyme gene, the iPS cells can be selected by the addition of a luminescent substrate. When the marker gene is a chromogenic enzyme gene, the iPS cells can be selected by the addition of a chromogenic substrate.

Alternatively, an established line of induced pluripotent stem cells may be obtained. For example, a human induced pluripotent stem cell line established by Kyoto University, such as 201B7 cells, 201B7-Ff cells, 253G1 cells, 253G4 cells, 1201C1 cells, 1205D1 cells, 1210B2 cells, or 1231A3 cells is available from Kyoto University and iPS Academia Japan, Inc. For example, Ff-I01 cells, Ff-I14 cells, and QHJI01s04 cells established by Kyoto University are available as established lines of induced pluripotent stem cells from Kyoto University. The iPS cells may be manufactured using, for example, somatic cells.

The term "somatic cells" used in the present specification refers to every animal cell (preferably mammalian (including human) cell) except for germline cells such as eggs, oocytes, and ES cells, or pluripotent stem cells. The somatic cells encompass, without limitations, all of fetal somatic cells, newborn somatic cells, and matured healthy or diseased somatic cells. The somatic cells also encompass all of primary cultured cells, subcultured cells, and established cell lines. Specific examples of the somatic cells include: (1) tissue stem cells (somatic stem cells) such as neural stem cells, hematopoietic stem cells, mesenchymal stem cells, and dental pulp stem cells; (2) tissue progenitor cells; and (3) differentiated cells such as lymphocytes, epithelial cells, endothelial cells, muscle cells, fibroblasts (skin cells, etc.), hair cells, hepatocytes, gastric mucosal cells, intestinal cells, splenocytes, pancreatic cells (exocrine pancreatic cells, etc.), brain cells, lung cells, kidney cells, and adipocytes.

In the case of using the iPS cells as a material of cells for transplantation, somatic cells having the same or substantially the same HLA genotype as that of a recipient individual is desirably used from the viewpoint of suppressing rejection after transplantation. In this context, the term "substantially the same" means that the HLA genotype is the same to the extent that immune response to the transplanted cells can be suppressed by an immunosuppressant. Such somatic cells have, for example, an HLA type showing a match of 3 gene loci of HLA-A, HLA-B, and HLA-DR or 4 gene loci of HLA-A, HLA-B, HLA-DR, and HLA-C.

### (E) ES cells derived from cloned embryo obtained by nuclear transfer

The somatic cell-derived ES cells (nt ES cells) are ES cells derived from a cloned embryo prepared by a nuclear transfer technique and have almost the same characteristics as those of fertilized egg-derived ES cells (T. Wakayama et al. (2001), Science, 292: 740-743; and S. Wakayama et al. (2005), Biol. Reprod., 72: 932-936; and J. Byrne et al. (2007), Nature, 450: 497-502). Specifically, the nt ES (nuclear transfer ES) cells are ES cells established from an inner cell mass of blastocyst derived from a cloned embryo obtained by replacing the nucleus of an unfertilized egg with the nucleus of a somatic cell. For the preparation of the nt ES cells, a nuclear transfer technique (J.B. Cibelli et al. (1998), Nature Biotechnol., 16: 642-646) and an ES cell preparation technique (supra) are used in combination (Kiyoka Wakayama et al.

(2008), Experimental Medicine, Vol. 26, No. 5 (extra edition), p. 47-52). In the nuclear transfer, the nuclei of somatic cells are injected to enucleated fertilized eggs of a mammal, which can then be cultured for several hours for reprogramming.

### (F) Multilineage-differentiating Stress Enduring cells (Muse cells)

The Muse cells are pluripotent stem cells manufactured by a method described in WO2011/007900. More specifically, the Muse cells are cells having pluripotency obtained by treating fibroblasts or bone marrow stromal cells with trypsin for a long time, preferably 8 hours or 16 hours, followed by suspension culture, and are SSEA-3- and CD105-positive cells.

### <Neural cells>

In the present specification, examples of the neural cells include every neural cell such as neural cells of the central nervous system, neural cells of the peripheral nervous system, neural cells of the autonomic nervous system, neural cells of the motor nervous system or the sensory system, neural cells of the midbrain floor plate region, and their stem cells or precursor cells.

### <Dopaminergic progenitor cells>

In the present specification, the dopaminergic progenitor cells mean precursor cells of dopamine-producing neuronal cells destined to differentiate into dopamine-producing neuronal cells (also called dopamine neurons or dopaminergic neurons). The dopaminergic progenitor cells are FOXA2-positive and βIII Tubulin (TUJ1)-positive and may preferably be cells further expressing (or cells positive to) one or more genes and proteins (differentiation markers) selected from the group consisting of OTX2, CORIN, CD142, LMX1A, LMX1B, EN1, Nurr1, PITX3, DAT, GIRK2, and TH. Cells expressing one type or a combination of plural types among these genes, the cells being capable of being induced to differentiate into dopamine-producing neuronal cells can be determined as the dopaminergic progenitor cells.

In the present specification, a cell population comprising the dopaminergic progenitor cells may comprise dopamine-producing neuronal cells. In the present specification, the cell population comprising the dopaminergic progenitor cells is preferably a cell population comprising no serotonergic neuron.

The cell population containing the dopaminergic progenitor cells is desirably a cell population containing cells expressing one or more proteins selected from the group consisting of FOXA2, βIII Tubulin (TUJ1), OTX2, CORIN, CD142, LMX1A, LMX1B, EN1, Nurr1, PITX3, DAT, GIRK2, and TH.

The dopaminergic progenitor cells can be induced by the differentiation of pluripotent stem cells under a mechanism common with development in living bodies, as mentioned later. First, neural progenitor cells are induced by the induction of pluripotent stem cells into neural cells (neural induction). A feature of the neural progenitor cells is, for example, to be SOX1-positive and/or NESTIN-positive.

The neural progenitor cells are then induced to differentiate into intermediate midbrain floor plate cells. Specifically, the neural progenitor cells are induced to differentiate into midbrain floor plate cells mentioned later via precursor cells of midbrain floor plate cells. The midbrain floor plate cells correspond to precursor cells of dopaminergic progenitor cells destined to differentiate into midbrain dopaminergic progenitor cells.

### <Neural cells of midbrain floor plate region>

The "midbrain floor plate region" or the "midbrain floor plate" is a region that appears only temporarily at the development stage of the mammalian fetal brain and spinal cord. Specifically, the fetal brain is classified into the forebrain, the midbrain, and the hindbrain along the anteroposterior axis, and the most ventral region along the dorsoventral axis is called floor plate. Dopamine-producing neuronal cells (also called dopaminergic neurons or dopamine neurons) are known to develop from the midbrain floor plate region. Specifically, in the case of inducing *in vitro* the differentiation of pluripotent stem cells into dopamine-producing neuronal cells or dopaminergic progenitor cells which are precursor cells thereof, these cells are also produced via neural cells of the midbrain floor plate region.

Specifically, in the present embodiment, the "neural cells of the midbrain floor plate region" are cells classified into neural cells among cells that appear in the midbrain floor plate region at the development stage, and mean cells capable of differentiating into dopaminergic progenitor cells and further dopaminergic neurons (dopamine-producing neuronal cells). The neural cells of the midbrain floor plate region encompass the midbrain floor plate cells mentioned above. In the present specification, the neural cells of the midbrain floor plate region are neural cells of the midbrain floor plate region induced by the differentiation of the pluripotent stem cells *in vitro.*

Examples of the neural cells of the midbrain floor plate region or the midbrain floor plate cells include cells positive to one or more genes and proteins selected from the group consisting of CORIN, LRTM1, LMX1A, FOXA2, NGN2, DDC, OTX2, LMX1B, and CD142. Specific examples thereof can include CORIN-positive cells, LRTM1-positive cells, FOXA2- and LMX1A-copositive cells, and cells positive to FOXA2 and further positive to one or more members selected from the group consisting of CORIN, LRTM1, NGN2, DDC, OTX2, LMX1A, LMX1B, and CD142.

In the present embodiment, a cell population comprising the neural cells of the midbrain floor plate region or the midbrain floor plate cells is preferably a population comprising FOXA2-positive cells and desirably further comprises, for example, cells expressing one or more genes and proteins selected from the group consisting of CORIN, LRTM1, OTX2, NGN2, DDC, LMX1A, LMX1B, and CD142.

The cell population comprising the neural cells of the midbrain floor plate region or the midbrain floor plate cells may comprise dopaminergic progenitor cells. In the present specification, the cell population comprising the neural cells of the midbrain floor plate region is desirably a cell population containing cells positive to one or more proteins selected from the group consisting of CORIN, LRTM1, FOXA2, LMX1A, LMX1B, and CD142.

### <Marker-positive cells>

In the present embodiment, the "marker-positive cells" mean cells expressing, on cell surface or intracellularly, a particular marker protein in an amount that allows the marker protein to be recognized by an antibody thereagainst.

In the present embodiment, the "Corin-positive cells" mean cells expressing, on cell surface, Corin protein in an amount that allows the protein to be recognized by an anti-Corin antibody. Examples of the Corin-positive cells include cells expressing, on cell surface, Corin protein in an amount that allows the cells to be recognized by a "method for selecting cells" mentioned later. Examples of the Corin-positive cells include midbrain floor plate cells and further include cells capable of differentiating into midbrain floor plate cells and dopaminergic progenitor cells under predetermined culture conditions.

### <Measuring concentration of NT-3>

This step comprises measuring a concentration of NT-3 in a culture supernatant in a culture broth obtained by culturing the pluripotent stem cells in a medium containing an inducer of differentiation into the neural cells of the midbrain floor plate region.

### (Concentration of NT-3)

In the present embodiment, the "NT-3" means neurotrophin-3, a neurotrophic factor. Examples of the method for measuring the concentration of NT-3 in a culture supernatant include, but are not particularly limited to, ELISA (Enzyme-Linked Immuno Sorbent Assay), chemiluminescent immunoassay (CLIA), latex agglutination, radioimmunoassay, immunoturbidimetry, enzyme activity measurement method, dye binding method, Western blotting, HumanMAP method, mass spectrometry, immunochromatography, and a method using a multiplex suspension array system. Examples of the multiplex suspension array system include Luminex(R) manufactured by Eurofins Genetic Lab Co., Ltd.

In one aspect of the present embodiment, the concentration of NT-3 in a culture supernatant may be measured by a measurement method with high detection sensitivity. Use of the measurement method with high detection sensitivity enables change in the concentration of NT-3 in a low-concentration range (e.g., 5 pg/ml to 20 pg/ml) to be detected sensitively and early. Examples of such a measurement method with high detection sensitivity include digital ELISA and ProQuantum High-Sensitive Immunoassays (Thermo Fisher Scientific).

In one aspect of the present embodiment, the method for measuring the concentration of NT-3 can be appropriately selected according to the cell line used, a manufacturing scale, and the purpose of evaluation, etc. A method of monitoring only elevation in the concentration of NT-3 or a method of monitoring the lowering of the concentration of NT-3 in addition to elevation in the concentration of NT-3 can be selected according to a specific manufacturing process. Change in the concentration of NT-3 in a manufacturing process is measured in advance, and a method compatible with each manufacturing process can be adopted.

The culture supernatant is a culture supernatant collected from the culture broth at any time from 48 hours to 240 hours after the start of culture in a medium containing an inducer of differentiation of the pluripotent stem cells (also referred to as "after the start of induction of differentiation") and is preferably a culture supernatant collected from the culture broth at any time from 48 hours to 192 hours after the start of culture of the pluripotent stem cells. In this context, the term "any time from 48 hours to 240 hours after the start of culture" is not particularly limited and is arbitrarily selected as long as NT-3 may be detected in the culture supernatant when differentiation into neural cells of the midbrain floor plate region progresses normally. Specifically, the timing of collecting the culture supernatant from the culture broth is any time from 48 hours to 240 hours after the start of culture and when NT-3 may be detected, and can be appropriately set according to a method for manufacturing individual cells of interest such as neural cells of the midbrain floor plate region or dopaminergic progenitor cells on the basis of a detectable concentration of NT-3 equal to or more than a reference concentration. In one aspect, the culture supernatant may be, for example, a culture supernatant collected from the culture broth at any time from 48 hours to 96 hours after the start of culture of the pluripotent stem cells. In another aspect of the present embodiment, the culture supernatant may be a culture supernatant collected from the culture broth at any time from 48 hours to 72 hours after the start of culture of the pluripotent stem cells. In an alternative aspect of the present embodiment, the culture supernatant may be a culture supernatant collected from the culture broth at any time from 120 hours to 192 hours after the start of culture (after the start of induction of differentiation) of the pluripotent stem cells. The number of times of collecting the culture supernatant is not particularly limited. In this context, the "start of culture in a medium containing an inducer of differentiation of the pluripotent stem cells" means the start of culture of the pluripotent stem cells in a medium that contains an inducer of differentiation into the neural cells of the midbrain floor plate region mentioned later and/or is placed under conditions that cannot maintain pluripotency without containing a factor necessary for the maintenance of pluripotency, such as bFGF. In one aspect, this phrase means the start of culture by replacing a medium for use in the culture (e.g., expansion culture) of the pluripotent stem cells with a medium for use in the induction of differentiation.

Medium replacement is performed, irrespective of whether or not to measure the concentration of NT-3, in order to properly perform cell culture in the differentiation induction step after the start of induction of differentiation. The frequency of medium replacement is not particularly limited as long as an inducer of differentiation and a nutrient necessary for the cells are properly replenished. The medium replacement is usually performed approximately every 12 hours, approximately every 24 hours, every 48 hours, or every 72 hours. For a medium replacement method, the whole amount of the medium may be replaced, or a portion (3/4, 1/2, 1/3, or 1/4 amount) of the medium may be replaced. In this context, in the case of continuing culture in a medium containing the same component as that in the preceding medium, medium replacement is generally performed by a given method through a period for which the culture is continued. Thus, the concentration of NT-3 contained in the culture supernatant is influenced by the frequency of medium replacement and a method for the replacement. Hence, the culture supernatant can be collected at a proper timing, though the concentration of NT-3 detected varies depending on an individual manufacturing process. Also, a proper reference concentration can be set on a manufacturing process basis. Specifically, in the case of setting a reference concentration, it is desirable to set the reference concentration according to the collection of the culture supernatant to be performed in a manufacturing process in consideration of the method and schedule of medium replacement.

The "concentration of NT-3" desirably correlates with the amount of NT-3 produced by the cells for a given period. For example, the concentration of NT-3 secreted from medium replacement before the time of collection to medium replacement at the time of collection is measured as long as the concentration is found in the culture supernatant collected by medium replacement that replaces the whole amount of the medium for a given period. This concentration means the "concentration of NT-3" described in the present specification. Thus, in the case of collecting a culture supernatant at the time of medium replacement, the concentration corresponds to the concentration of NT-3 in a culture supernatant discarded by medium replacement operation. For example, the "concentration of NT-3" in a liquid waste of the culture supernatant in a medium replaced every 24 hours refers to the "concentration of NT-3" that corresponds to the amount of NT-3 secreted by the cells into the culture supernatant from the time of the preceding medium replacement to after a lapse of 24 hours which is the timing of the subsequent medium replacement.

In one aspect, when a portion of the culture supernatant in a medium without medium replacement is sampled, i.e., when no medium replacement is performed between two times of sampling, a cumulative amount of NT-3 secreted by the cells contained in the culture broth is measured. Thus, the difference in the concentration of NT-3 between two times of sampling is calculated and thereby corresponds to the "concentration of NT-3" in the second sampling.

It is no problem to replace the whole amount of the medium and collect a liquid waste because the whole medium is stirred. In the case of collecting a portion of the medium from a culture vessel, it is desirable to predetermine the collection position or to collect the culture supernatant after rendering the NT-3 concentration uniform by gentle stirring of the medium, in consideration of the possibility that the concentration of NT-3 differs depending on the distance between the cells and the sampling position of the culture supernatant.

The "culture supernatant" means a liquid component in a culture broth containing cells such as pluripotent stem cells and a medium. In one aspect of the present embodiment, the culture supernatant may be interpreted as the culture broth from which the cells have been removed. In one aspect of the present embodiment, the culture supernatant may be interpreted as a culture supernatant at a stage where the induction of differentiation into neural cells (by a SMAD inhibitor) and ventralization (by SHH mentioned later) are performed. In one aspect of the present embodiment, the culture supernatant may be interpreted as a culture supernatant at a stage where the induction of differentiation into neural cells (by a SMAD inhibitor) and ventralization (by SHH mentioned later) are performed, and 24 hours before to 24 hours after posteriorization (by an increased WNT signal mentioned later). In an alternative aspect of the present embodiment, the culture supernatant may be interpreted as a culture supernatant at a stage where the induction of differentiation into neural cells (by a SMAD inhibitor) and ventralization (by SHH mentioned later) are performed, and 24 hours before to 120 hours after posteriorization (by an increased WNT signal mentioned later). In an alternative aspect of the present embodiment, the culture supernatant may be interpreted as a culture supernatant at a stage where the induction of differentiation into neural cells (by a SMAD inhibitor) is started, and ventralization (by SHH mentioned later) and/or posteriorization (by an increased WNT signal mentioned later) progresses.

In the case of performing adhesion culture or the culture of cell aggregates, the culture supernatant can be collected from the culture broth using a pipette or the like without being centrifuged. Alternatively, a culture supernatant discarded at the time of medium replacement may be collected.

### (Medium)

In the present embodiment, the medium for inducing the differentiation of pluripotent stem cells into neural cells of the midbrain floor plate region contains an inducer of differentiation into the neural cells of the midbrain floor plate region. The medium can be prepared with a medium for use in animal cell culture as a basal medium. The basal medium encompasses, for example, Glasgow's Minimal Essential Medium (GMEM), Iscove's Modified Dulbecco's Medium (IMDM), Medium 199, Eagle's Minimum Essential Medium (EMEM), αMEM medium, Dulbecco's modified Eagle's Medium (DMEM), Ham's F12 medium, RPMI1640 medium, Fischer's medium, Neurobasal Medium (Life Technologies Corp.), and mixed media thereof. The basal medium is preferably GMEM medium. The medium may or may not contain serum. The medium containing no serum is also referred to as a serum-free medium. The medium may optionally contain, for example, one or more serum replacements such as albumin, transferrin, Knockout Serum Replacement (KSR) (serum replacement of FBS for ES cell culture), N2 supplement (Invitrogen), B27 supplement (Invitrogen), fatty acid, insulin, procollagen, trace element, 2-mercaptoethanol, and 3'-thiolglycerol. The medium may contain one or more substances such as a lipid, an amino acid, L-glutamine, Glutamax (Invitrogen), nonessential amino acids, a vitamin, a growth factor, a low-molecular compound, an antibiotic, an antioxidant, pyruvic acid, a buffer, and inorganic salts. The medium is preferably GMEM medium containing KSR, 2-mercaptoethanol, nonessential amino acids, and pyruvic acid. This medium is properly supplemented with one reagent or a combination of two or more reagents (i.e., an inducer of differentiation) appropriately selected from the group consisting of a BMP signaling-inhibiting substance, a TGFβ signaling-inhibiting substance, a SHH signaling-activating substance, FGF8 (fibroblast growth factor 8), and a Wnt signaling-activating substance (e.g., a GSK-3β-inhibiting substance) mentioned later, and the resulting one or more types of media can be sequentially used in combination in a proper order in culture.

### (Inducer of differentiation into neural cells of midbrain floor plate region)

In the present embodiment, the "inducer of differentiation into the neural cells of the midbrain floor plate region" means a drug (substance) that induces the differentiation of pluripotent stem cells into neural cells of the midbrain floor plate region, or a combination of the drugs. Specifically, the "inducer of differentiation into the neural cells of the midbrain floor plate region" also conceptually includes a combination of a drug necessary for inducing the differentiation of pluripotent stem cells into neural cells of the midbrain floor plate region, and the "timing of using a medium containing the drug". The inducer of differentiation into the neural cells of the midbrain floor plate region is not particularly limited, and, for example, an inducer of differentiation used in a method for inducing differentiation described in NPL 2 (Frontiers in Cell and Developmental Biology, August 2020, Volume 8, Article 729) can be used. As one example, culture conditions involving a SMAD signaling-inhibiting substance can be used, and culture conditions involving at least one type of SMAD signaling-inhibiting substance are preferred. In other words, the inducer of differentiation into the neural cells of the midbrain floor plate region preferably comprises at least one SMAD signaling-inhibiting substance. As one example, in the case of using two or more types of SMAD signaling-inhibiting substances, a medium containing all the SMAD signaling-inhibiting substances used may be used in culture, or media each containing some (or one) of the SMAD signaling-inhibiting substances used may be combined over time in culture. In other words, all the SMAD signaling-inhibiting substances used may be added at the same time to the medium, or each of the SMAD signaling-inhibiting substances used may be sequentially added to the medium at a proper timing.

In the present embodiment, the "SMAD signaling-inhibiting substance" means a substance that inhibits signaling mediated by a transcriptional factor SMAD (Small Mothers against Decapentaplegic). The SMAD signaling-inhibiting substance preferably comprises at least one BMP signaling-inhibiting substance and at least one TGFβ signaling-inhibiting substance.

In the present embodiment, the "BMP signaling-inhibiting substance" means a substance that inhibits signaling mediated by a signal protein BMP (bone morphogenetic protein). Examples of the BMP signaling-inhibiting substance include inhibitor proteins such as Chordin, Noggin, and Follistatin, Dorsomorphin (i.e., 6-[4-(2-piperidin-1-yl-ethoxy)phenyl]-3-pyridin-4-yl-pyrazolo[1,5-a]pyrimidine), derivatives thereof (P.B. Yu et al. (2007), Circulation, 116: II_60; P.B. Yu et al. (2008), Nat. Chem. Biol., 4: 33-41; and J. Hao et al. (2008), PLoS ONE, 3 (8): e2904) and LDN-193189 (i.e., 4-(6-(4-(piperazin-1-yl)phenyl)pyrazolo[1,5-a]pyrimidin-3-yl)quinoline), DMH1, K02288, LDN-214117, LDN-212854, and ML347 (LDN193719). Dorsomorphin and LDN-193189 are commercially available and may be obtained from Sigma-Aldrich Co. LLC and Stemgent, Inc., respectively. The BMP signaling-inhibiting substance preferably comprises at least one member selected from the group consisting of LDN-193189, Noggin, DMH1, Chordin, Follistatin, K02288, LDN-214117, LDN-212854, ML347 (LDN193719), and Dorsomorphin.

The concentration of LDN-193189 in the culture broth is not particularly limited as long as the concentration inhibits BMP. The concentration is, for example, 1 nM to 50 µM, preferably 1 nM to 1000 nM, more preferably 50 nM to 300 nM, more specifically 1 nM, 10 nM, 50 nM, 100 nM, 500 nM, 750 nM, 1 µM, 2 µM, 3 µM, 4 µM, 5 µM, 6 µM, 7 µM, 8 µM, 9 µM, 10 µM, 15 µM, 20 µM, 25 µM, 30 µM, 40 µM, or 50 µM, though the concentration is not limited thereto.

In the case of using other substances as the BMP signaling-inhibiting substance, a concentration that corresponds to the concentration of LDN-193189 mentioned above and exhibits BMP signaling inhibitory activity can be appropriately adopted.

In the present embodiment, the "TGFβ signaling-inhibiting substance" means a substance that inhibits signaling mediated by a growth factor TGFβ (Transforming Growth Factor-β). Examples of the TGFβ signaling-inhibiting substance include substances that inhibit binding to the receptor ALK family, and substances that inhibit the phosphorylation of SMAD by the ALK family, and include Lefty-1 (NCBI Accession No.: e.g., mouse : NM_010094, human : NM_020997), Lefty-2, SB-431542, and SB-202190 (all described in R.K. Lindemann et al., Mol. Cancer, 2003, 2: 20), SB-505124 (GlaxoSmithKline), NPC30345, SD093, SD908, SD208 (Scios), LY2109761, LY364947, LY580276 (Lilly Research Laboratories), A-83-01 (WO2009/146408), Galunisertib (LY2157299), SB-525334, Prifenidone (S-7701), GW788388, E-616452 (RepSox), TP0427736, BIBF-0775, LY3200882, Vactosertib (TEW-7197), ITD-1, and derivatives thereof. The TGFβ signaling-inhibiting substance preferably comprises at least one member selected from the group consisting of SB-431542, A-83-01, Lefty-1, Lefty-2, Galunisertib (LY2157299), SB-202190, SB-525334, SB-505124, NPC30345, Prifenidone (S-7701), GW788388, E-616452 (RepSox), SD208, TP0427736, BIBF-0775, LY3200882, Vactosertib (TEW-7197), ITD-1, SD093, SD908, LY2109761, LY364947, and LY580276, and more preferably comprises at least one member selected from the group consisting of SB-431542, A-83-01, Lefty-1, Lefty-2, Galunisertib (LY2157299), SB-202190, SB-525334, SB-505124, NPC30345, Prifenidone (S-7701), GW788388, E-616452 (RepSox), SD208, ITD-1, SD093, SD908, LY2109761, LY364947, and LY580276.

In one aspect of the present embodiment, preferably, the BMP signaling-inhibiting substance comprises at least one member selected from the group consisting of LDN-193189, Noggin, DMH1, Chordin, Follistatin, K02288, LDN-214117, LDN-212854, ML347, and Dorsomorphin, and the TGFβ signaling-inhibiting substance comprises at least one member selected from the group consisting of SB-431542, A-83-01, Lefty-1, Lefty-2, Galunisertib (LY2157299), SB-202190, SB-525334, SB-505124, NPC30345, Prifenidone (S-7701), GW788388, E-616452(RepSox), SD208, TP0427736, BIBF-0775, LY3200882, Vactosertib (TEW-7197), ITD-1, SD093, SD908, LY2109761, LY364947, and LY580276.

The concentration of A-83-01 in the culture broth is not particularly limited as long as the concentration inhibits ALK5. The concentration is, for example, 1 nM to 100 µM, preferably 100 nM to 5 µM, more preferably 300 nM to 1 µM, more specifically 1 nM, 10 nM, 50 nM, 100 nM, 500 nM, 750 nM, 1 µM, 2 µM, 3 µM, 4 µM, 5 µM, 6 µM, 7 µM, 8 µM, 9 µM, 10 µM, 15 µM, 20 µM, 25 µM, 30 µM, 40 µM, or 50 µM, though the concentration is not limited thereto.

In the case of using other substances as the TGFβ signaling-inhibiting substance, a concentration that corresponds to the concentration of A-83-01 mentioned above and exhibits TGFβ signaling inhibitory activity can be appropriately adopted.

### (SHH signaling-activating substance and Wnt signaling-activating substance)

In one aspect of the present embodiment, the medium preferably further contains a SHH signaling-activating substance and a Wnt signaling-activating substance.

The "SHH signaling-activating substance" means a substance capable of enhancing signaling mediated by SHH (Sonic hedgehog). Examples of the SHH signaling-activating substance include proteins belonging to the Hedgehog family (e.g., SHH and IHH) and fragments, modified forms, and mutants thereof, SHH receptors, SHH receptor agonists, Hh-Ag1.5 (Li, X., et al., Nature Biotechnology, 23, 215-221 (2005)), Smoothened Agonist, SAG (N-Methyl-N'-(3-pyridinylbenzyl)-N'-(3-chlorobenzo[b]thiophene-2-carbonyl)-1,4-diaminocyclohexane), 20a-hydroxycholesterol, Purmorphamine, and derivatives thereof (Stanton BZ, Peng LF., Mol Biosyst. 6: 44-54, 2010). The SHH signaling-activating substance preferably comprises at least one member selected from the group consisting of SHH and a fragment thereof (e.g., Shh (C24II) N-Terminus and Shh (C25II) N-Terminus) and a modified form, a SHH receptor, a SHH receptor agonist, Hh-Ag1.5, Smoothened Agonist, 20a-hydroxycholesterol, Purmorphamine, and SAG.

As one example, the SMAD signaling-inhibiting substance and the SHH signaling-activating substance can be used in combination. In the case of using these drugs in combination, a medium containing all the drugs used may be used in culture, or media each containing some (or one) of the drugs used may be combined over time in culture. In other words, all the drugs used may be added at the same time to the medium, or each of the drugs used may be sequentially added to the medium at a proper timing.

The concentration of Purmorphamine in the culture broth is not particularly limited as long as the concentration activates Gli2. The concentration is, for example, 1 nM to 50 µM, preferably 100 nM to 10 µM, more preferably 500 nM to 5 µM, more specifically 1 nM, 10 nM, 50 nM, 100 nM, 500 nM, 750 nM, 1 µM, 2 µM, 3 µM, 4 µM, 5 µM, 6 µM, 7 µM, 8 µM, 9 µM, 10 µM, 15 µM, 20 µM, 25 µM, 30 µM, 40 µM, or 50 µM, though the concentration is not limited thereto. In the case of using other substances as the SHH signaling-activating substance, a concentration that corresponds to the concentration of Purmorphamine mentioned above and exhibits SHH signaling activity can be appropriately adopted.

The "Wnt signaling-activating substance" means a substance that activates signaling resulting from the binding of Wnt protein to a Frizzled receptor. The Wnt signaling-activating substance preferably comprises at least one member selected from the group consisting of WNT3A, and a GSK-3β-inhibiting substance.

The "GSK-3β-inhibiting substance" means a substance that inhibits the kinase activity (e.g., ability to phosphorylate β catenin) of GSK-3β protein. Examples of the GSK-3β-inhibiting substance include an indirubin derivative BIO (also called GSK-3β inhibitor IX;6-bromoindirubin 3'-oxime), a maleimide derivative SB216763 (3-(2,4-dichlorophenyl)-4-(1-methyl-1H-indol-3-yl)-1H-pyrrole-2,5-dione), a phenyl α-bromomethyl ketone compound GSK-3β inhibitor VII (4-dibromoacetophenone), a cell-penetrating phosphorylated peptide L803-mts (also called GSK-3β peptide inhibitor; Myr-N-GKEAPPAPPQpSP-NH₂ (SEQ ID NO: 1)) and CHIR99021 having high selectivity (6-[2-[4-(2,4-Dichlorophe nyl)-5-(4-methyl-1H-imidazol-2-yl)pyrimidin-2-ylamino]ethylamino]pyridine-3-carbonitrile), CHIR98014, and SKL2001. These compounds are commercially available from, for example, Calbiochem or Biomol GmbH and may be readily utilized. Such a compound may be obtained from other distributors or may be prepared per se. The GSK-3β-inhibiting substance preferably comprises at least one member selected from the group consisting of CHIR99021, BIO, CHIR98014, SKL2001, SB216763, GSK-3β inhibitor VII (4-dibromoacetophenone), and L803-mts.

The concentration of CHIR99021 in the culture broth is, for example, 1 nM to 50 µM, preferably 10 nM to 20 µM, more preferably 100 nM to 10 µM, more specifically 1 nM, 10 nM, 50 nM, 100 nM, 500 nM, 750 nM, 1 µM, 2 µM, 3 µM, 4 µM, 5 µM, 6 µM, 7 µM, 8 µM, 9 µM, 10 µM, 15 µM, 20 µM, 25 µM, 30 µM, 40 µM, or 50 µM, though the concentration is not limited thereto. In the case of using other substances as the Wnt signaling-activating substance or the GSK3β-inhibiting substance, a concentration that corresponds to the concentration of CHIR99021 mentioned above and exhibits Wnt signaling activity or GSK3β inhibitory activity can be appropriately adopted.

As one example, the Wnt signaling-activating substance or the GSK3β-inhibiting substance, the SMAD signaling-inhibiting substance, and the SHH signaling-activating substance can be used in combination. In this case, it is not necessary to add all of these drugs at the same time to the medium. These drugs may be appropriately combined over time in culture such that differentiation can be induced. In other words, each of the drugs used may be sequentially added to the medium at a proper timing.

One example of such an approach includes a method of increasing or decreasing the types of drugs contained in a medium over time, comprising (1) performing culture in a medium containing one or more, preferably two or more SMAD signaling-inhibiting substances, (2) further performing culture in a medium containing the SHH signaling-activating substance, and then (3) further performing culture in a medium containing the Wnt signaling-activating substance.

In one aspect of the present embodiment, the medium preferably contains FGF8 (fibroblast growth factor 8). Examples of the FGF8 include, but are not particularly limited to, four splicing forms FGF8a, FGF8b, FGF8e, and FGF8f for human FGF8. FGF8b is more preferred. The FGF8 is commercially available from, for example, FUJIFILM Wako Pure Chemical Corp. or R&D systems, Inc. and may be readily utilized. The FGF8 may be obtained by forced expression in cells by a method known to those skilled in the art.

As one example, the FGF8, the SMAD signaling-inhibiting substance, the SHH signaling-activating substance, and the Wnt signaling-activating substance or the GSK3β-inhibiting substance can be used in combination. In this case, it is not necessary to add all of these drugs at the same time to the medium. These drugs may be appropriately combined over time in culture such that differentiation can be induced. In other words, each of the drugs used may be sequentially added to the medium at a proper timing.

The concentration of FGF8 in the culture broth is, for example, 1 ng/mL to 5000 ng/mL, preferably 10 ng/mL to 1000 ng/mL, more specifically, 1 ng/mL, 5 ng/mL, 10 ng/mL, 50 ng/mL, 100 ng/mL, 150 ng/mL, 200 ng/mL, 250 ng/mL, 500 ng/mL, 1000 ng/mL, 2000 ng/mL, or 5000 ng/mL, though the concentration is not limited thereto.

### <Comparing measured concentration of NT-3 with reference concentration>

This step comprises comparing the measured concentration of NT-3 with a reference concentration. The "reference concentration" is the concentration of NT-3 (cutoff value) serving as a reference for assessing whether the subject pluripotent stem cells are capable of differentiating into neural cells of the midbrain floor plate region, and can be arbitrarily set. For example, an arbitrary value including a value of the detection limit can be adopted as the reference concentration as long as the value can be statistically discriminated from the detection limit value. The reference concentration may be the concentration of NT-3 at which the pluripotent stem cells have been cultured under predetermined conditions and successfully differentiated into neural cells of the midbrain floor plate region, or may be a preset concentration. The preset concentration can be set, for example, as follows: first, pluripotent stem cells are cultured using a medium and culture conditions that allow for induction of differentiation, i.e., in accordance with documented manufacturing procedures (protocol) for neural cells of the midbrain floor plate region, and the NT-3 concentration in the culture supernatant is measured when cells of interest that achieve a goal are obtained, i.e., when differentiation induction efficiency is favorable. Such culture and measurement are performed a plurality of times (at least twice, preferably at least five times), and an average value of the respectively measured NT-3 concentrations can be set as the reference concentration. Alternatively, pluripotent stem cells are cultured using a medium and culture conditions that allow for induction of differentiation, and a concentration that offers sensitivity and specificity necessary for a user can be set as the reference concentration on the basis of the relationship between the reference concentration and sensitivity and specificity estimated from a ROC curve derived from the concentration of NT-3 in the culture supernatant when cells of interest that achieve a goal are obtained, and the concentration of NT-3 in the culture supernatant when the cells of interest that achieve a goal are not obtained, i.e., when differentiation induction efficiency is poor.

In this context, the phrase "when cells of interest that achieve a goal are obtained" or "when differentiation induction efficiency is favorable" described in the present specification means that the cells of interest are obtained at a level equal to or more than an amount (percentage) compatible with the goal, i.e., the performance of manufacturing. The "amount (percentage) compatible with the goal" can be appropriately set and is, for example, 20% or more, 30% or more, 40% or more, 50% or more, 60% or more, 70% or more, 80% or more, or 90% or more based on all the cells cultured.

The phrase "when the cells of interest that achieve a goal are not obtained" or "when differentiation induction efficiency is poor" described in the present specification means that the cells of interest are not obtained in the amount (percentage) compatible with the goal even though the induction of differentiation is performed in accordance with documented manufacturing procedures (protocol) for dopaminergic progenitor cells, or neural cells of the midbrain floor plate region which are a cell population of their intermediate midbrain floor plate cells. "Incompatibility" results showing that the cells of interest are not obtained in the amount compatible with the goal are obtained at least twice, preferably at least five time, and an average value of the respectively measured NT-3 concentrations is desirably set as the "concentration of NT-3 in the culture supernatant when differentiation induction efficiency is poor".

Alternatively, the phrase "when the cells of interest that achieve a goal are not obtained" or "when differentiation induction efficiency is poor" means that, for example, culture is performed using a medium or culture conditions inferior in differentiation induction efficiency on purpose in order to set a reference value, i.e., an "incompatibility" model case is established in which the cells of interest are not obtained in the amount compatible with the goal. Such an incompatibility model case means that the induction of differentiation is performed under conditions partially deviated on purpose from conditions stipulated in the documented manufacturing procedures as conditions reportedly proper for obtaining the cells of interest, i.e., required to be properly managed in the documented manufacturing procedures (protocol) for dopaminergic progenitor cells, or neural cells of the midbrain floor plate region or dopaminergic progenitor cells which are a cell population of their intermediate midbrain floor plate cells. In this case, "incompatibility" results showing that the cells of interest are not obtained in the amount compatible with the goal are obtained at least twice, preferably at least five time, by the induction of differentiation deviated from the stipulated conditions, and an average value of the respectively measured NT-3 concentrations is desirably set as the "concentration of NT-3 in the culture supernatant when differentiation induction efficiency is poor". In this context, the "conditions partially deviated from conditions stipulated in the documented manufacturing procedures" mean conditions under which the induction of differentiation into the cells of interest progresses properly and however, cannot reach the amount compatible with the goal. Conditions under which the induction of differentiation into the cells of interest does not occur in the first place, or conditions that markedly influence the induction of differentiation into the cells of interest are not proper for the model case performed for the purpose of setting the reference value. Such conditions are excluded from the "conditions partially deviated from conditions stipulated in the documented manufacturing procedures" for the purpose of determining the reference value. For example, culture conditions under which the ratio of the cells of interest to all the cells cultured is always 15% or less, 10% or less, or 5% or less are desirably excluded from the "conditions partially deviated from conditions stipulated in the documented manufacturing procedures" mentioned above. For example, culture conditions that offer 20% or less (15% or less, 10% or less, or 5% or less) based on the ratio of the cells of interest to all the cells set as the "amount (percentage) compatible with the goal" mentioned above are also desirably excluded from the "conditions partially deviated from conditions stipulated in the documented manufacturing procedures" mentioned above.

In this context, examples of the conditions required to be properly managed in the documented manufacturing procedures (protocol) include culture conditions capable of influencing the production efficiency of the cells of interest on the premise that a basic method for inducing differentiation is maintained, and include culture conditions such as medium components, the freshness of the medium, a medium temperature, medium pH, and a carbon dioxide concentration, the handling (manipulation such as pipetting or separation) of cells with less physical stress, no bacterial or viral contamination, and a method for and the frequency of passages or medium replacement.

The proper timing of detecting the NT-3 concentration can be appropriately set such that the pluripotent stem cells are cultured using a medium and culture conditions that allow for induction of differentiation, i.e., cultured in accordance with documented manufacturing procedures (protocol) for neural cells of the midbrain floor plate region or dopaminergic progenitor cells, and the NT-3 concentration can be effectively measured. For example, culture and measurement are performed a plurality of times (e.g., twice, preferably five times) over time, and a timing at which the NT-3 concentration can be detected can be appropriately set.

In one aspect, a partial liquid waste is collected at the time of medium replacement, and the NT-3 concentration may be measured. Proper measurement frequency can be appropriately set. The NT-3 concentration may be measured, for example, on a medium replacement basis.

The reference concentration is preferably set, specifically, to one point between 0.1 pg/ml or more and 10000 pg/ml or less, more preferably one point between 0.3 pg/ml or more and 3000 pg/ml or less, further specifically between 0.3 pg/ml and 100 pg/ml.

In one aspect of the present embodiment, the concentration of NT-3 before the amount of NT-3 secreted increases may be used as the reference concentration. For example, the concentration of NT-3 measured within 48 hours, preferably within 24 hours, after the start of induction of differentiation may be used as the reference concentration.

### <Assessing cells in culture broth as capable of differentiating into neural cells of midbrain floor plate region>

This step comprises, when the concentration of NT-3 is equal to or more than the reference concentration, assessing the cells (e.g., pluripotent stem cells and cells derived from the pluripotent stem cells) in the culture broth at the time when the concentration of NT-3 has been measured, as capable of differentiating into the neural cells of the midbrain floor plate region, i.e., as having differentiation potential into the neural cells of the midbrain floor plate region, and further having differentiation potential into dopaminergic progenitor cells. In one aspect of the present embodiment, the phrase "capable of differentiating" may be interpreted as "likely to differentiate".

In one aspect of the present embodiment, the phrase "capable of differentiating" may mean that the neural cells of the midbrain floor plate region are obtained in a targeted amount in a differentiation induction step in the manufacturing of the neural cells of the midbrain floor plate region. In one aspect of the present embodiment, the phrase "assessing as capable of differentiating" refers to determining that a differentiation induction step progresses smoothly in managing the step in the manufacturing of the neural cells of the midbrain floor plate region.

In one aspect of the present embodiment, the case of estimating that the neural cells of the midbrain floor plate region are not obtained in a targeted amount in a differentiation induction step in the manufacturing of the neural cells of the midbrain floor plate region corresponds to the case where the cells "cannot be assessed as capable of differentiating".

The "concentration of NT-3" used in the present invention can be applied not only to assessing the cells in the culture broth as capable of differentiating into the neural cells of the midbrain floor plate region, but to assessing, at an initial stage of a manufacturing process, whether or not to satisfy required quality and amount as intermediate cells for manufacturing "dopaminergic progenitor cells serving as an active pharmaceutical ingredient at a level (grade) that enables the cells to be used as a cell medicament in treatment".

According to the method of the present invention, it can be determined early at an initial stage of induction of differentiation of pluripotent stem cells into dopaminergic progenitor cells that the cells are properly destined to differentiate into the cells of interest. Furthermore, the assessing method of the present invention is very useful in managing a manufacturing process. Moreover, it can also be determined by use of the assessing method of the present invention that a cell line of pluripotent stem cells is suitable for the induction of differentiation into the cells of interest. In the case of optimizing conditions for the scale-up of manufacturing, the assessing method of the present invention can also be used for assessing differentiation induction efficiency.

In one aspect of the present embodiment, the phrase "the concentration of NT-3 is equal to or more than the reference concentration" conceptually includes the case where the concentration of NT-3 is equivalent to the reference concentration, and the case where the concentration of NT-3 is higher than the reference concentration. For example, when the concentration of NT-3 is 1 to 1000 times the reference concentration, the cells in the culture broth are preferably assessed as capable of differentiating into the neural cells of the midbrain floor plate region. When the concentration of NT-3 is 1.2 to 1000 times the reference concentration, the cells contained in the culture broth at the time when the concentration of NT-3 has been measured are more preferably assessed as capable of differentiating into the neural cells of the midbrain floor plate region.

The ratio at which the cell population assessed as capable of differentiating into the neural cells of the midbrain floor plate region by the assessing method of the present invention differentiates into the neural cells of the midbrain floor plate region may vary depending on culture conditions and the pluripotent stem cells used. The cells are considered to differentiate into the neural cells of the midbrain floor plate region at a ratio of, for example, 20% or more, preferably 30% or more, more preferably 40% or more, further preferably 50% or more, 60% or more, 70% or more, 80% or more, or 90% or more, based on all the cells cultured.

In one aspect of the present embodiment, when the concentration of NT-3 is less than the reference concentration, the cells in the culture broth or the culture broth itself may be discarded from the viewpoint of efficiently inducing differentiation. It is desirable to appropriately change the determination of whether to perform the discarding and the timing of the discarding according to a manufacturing method, a culture scale, the type and line of the cells used, a finally necessary amount of the cells, the targeted grade, the presence or absence of preculture and the status of progression thereof, the whole manufacturing schedule, the presence or absence of other determination methods, the number of days required for the determination, and cost, etc.

### <<Method for assessing differentiation potential of cells in culture broth in differentiation of pluripotent stem cells into neural cells of midbrain floor plate region - (2)>>

The second method for assessing differentiation potential of cells in a culture broth in the differentiation of pluripotent stem cells into neural cells of the midbrain floor plate region according to the present embodiment is
a method for assessing differentiation potential of cells in a culture broth in the differentiation of pluripotent stem cells into neural cells of the midbrain floor plate region, comprising:
measuring a concentration of NT-3 in each of a first culture supernatant and a second culture supernatant in a culture broth obtained by culturing the pluripotent stem cells in a medium containing an inducer of differentiation into the neural cells of the midbrain floor plate region;
determining a rate of change in the concentration of NT-3 from the concentration of NT-3 in the first culture supernatant and the concentration of NT-3 in the second culture supernatant;
comparing an absolute value of the rate of change in the concentration of NT-3 with a reference rate of change; and
when the absolute value of the rate of change in the concentration of NT-3 is equal to or more than the reference rate of change, assessing the cells in the culture broth as capable of differentiating into the neural cells of the midbrain floor plate region, wherein
the first culture supernatant is a culture supernatant collected from the culture broth at any time from 48 hours to 192 hours after the start of culture of the pluripotent stem cells, and
the second culture supernatant is a culture supernatant collected from the culture broth at any time from 24 hours to 96 hours before collection of the first culture supernatant or at any time from 24 hours to 96 hours after collection of the first culture supernatant. Hereinafter, detailed description will be made. The cells described in the first method mentioned above may be used as the pluripotent stem cells.

### <Measuring concentration of NT-3>

This step comprises measuring a concentration of NT-3 in each of a first culture supernatant and a second culture supernatant in a culture broth obtained by culturing the pluripotent stem cells in a medium containing an inducer of differentiation into the neural cells of the midbrain floor plate region. The method for measuring the concentration can adopt the measurement method described in the first method mentioned above.

The first culture supernatant is a culture supernatant collected from the culture broth at any time from 48 hours to 192 hours after the start of culture of the pluripotent stem cells. In one aspect, the first culture supernatant may be a culture supernatant collected from the culture broth at any time from 48 hours to 96 hours after the start of culture of the pluripotent stem cells. In another aspect, the first culture supernatant may be a culture supernatant collected from the culture broth at any time from 48 hours to 72 hours after the start of culture of the pluripotent stem cells.

In one aspect, the second culture supernatant is collected from the culture broth at the start of differentiation or at a later timing and before collection of the first culture supernatant, for example, 24 hours to 96 hours (preferably 24 hours to 72 hours) after the start of differentiation and before collection of the first culture supernatant. Specifically, examples thereof include any time from 24 hours to 96 hours (preferably 24 hours to 72 hours) before collection of the first culture supernatant.

In one aspect, the second culture supernatant is collected from the culture broth after collection of the first culture supernatant. Specifically, examples thereof include any time from 24 hours or later after collection of the first culture supernatant to the completion of differentiation into the neural cells of the midbrain floor plate region.

In this context, the "completion of differentiation into the neural cells of the midbrain floor plate region" means a state where cell differentiation progresses to the extent that a marker of the neural cells of the midbrain floor plate region is expressed. In this respect, neural cells into which the neural cells of the midbrain floor plate region further differentiate may be contained in the culture broth. Specific examples thereof include a differentiation stage at which a plurality of markers of the neural cells of the midbrain floor plate region are detected. Examples of the markers can include Corin, Foxa2, Lmx1a, Lmx1b, and CD142.

The second culture supernatant is preferably a culture supernatant collected from the culture broth at any time from 24 hours to 48 hours before collection of the first culture supernatant or at any time from 24 hours to 96 hours (more preferably 24 hours to 72 hours) after collection of the first culture supernatant. In this context, the phrase "any time from 24 hours to 96 hours before collection of the first culture supernatant" means any time in a period between 24 hours before the time when the first culture supernatant is collected and 96 hours before the time when the first culture supernatant is collected.

The composition of the medium for culturing the pluripotent stem cells can adopt the composition described in the first method mentioned above.

### <Determining rate of change in concentration of NT-3>

This step comprises determining a rate of change in the concentration of NT-3 from the concentration of NT-3 in the first culture supernatant and the concentration of NT-3 in the second culture supernatant. The rate of change in the concentration of NT-3 can be determined according to the following expressions.

When the second culture supernatant is collected earlier than the first culture supernatant (Rate of change in the concentration of NT-3) = {(Concentration of NT-3 in the first culture supernatant) - (Concentration of NT-3 in the second culture supernatant)}/ {(Time when the first culture supernatant is collected) - (Time when the second culture supernatant is collected)}

When the second culture supernatant is collected later than the first culture supernatant (Rate of change in the concentration of NT-3) = {(Concentration of NT-3 in the second culture supernatant) - (Concentration of NT-3 in the first culture supernatant)}/ {(Time when the second culture supernatant is collected) - (Time when the first culture supernatant is collected)}

In one aspect of the present embodiment, the concentration of NT-3 in the culture supernatant may increase temporarily in a period up to 12 days from the start of differentiation (period up to 288 hours after the start of culture of the pluripotent stem cells) and decrease in 24 hours or 48 hours. In another aspect of the present embodiment, the concentration of NT-3 in the culture supernatant may increase temporarily in a period up to 12 days from the start of differentiation, and the period of the increased concentration may continue for approximately 168 hours. In this context, the "period of the increased concentration" means a period for which the concentration of NT-3 is higher than the baseline value. A temporarily constant or decreased concentration of NT-3 is also included in the "period of the increased concentration" as long as the concentration is higher than the baseline value.

In one aspect of the present embodiment, the concentration of NT-3 in the culture supernatant may increase continuously in a period up to 12 days from the start of differentiation (period up to 288 hours after the start of culture of the pluripotent stem cells).

The timings of collecting the first culture supernatant and the second culture supernatant can be appropriately set according to a feature of change in the concentration of NT-3 in each differentiation induction step.

### <Comparing absolute value of rate of change in concentration of NT-3 with reference rate of change>

This step comprises comparing an absolute value of the rate of change in the concentration of NT-3 with a reference rate of change. The "reference rate of change" is the absolute value of the rate of change in the concentration of NT-3 (cutoff value) serving as a reference for assessing whether the subject pluripotent stem cells are capable of differentiating into neural cells of the midbrain floor plate region, and can be arbitrarily set. The reference rate of change may be the absolute value of the rate of change in the concentration of NT-3 at which the pluripotent stem cells known beforehand to differentiate into neural cells of the midbrain floor plate region have been cultured under predetermined conditions, or may be the absolute value of a preset rate of change. The absolute value of the preset rate of change can be set, for example, as follows: first, pluripotent stem cells are cultured using a medium and culture conditions that allow for induction of differentiation, i.e., in accordance with documented manufacturing procedures (protocol) for neural cells of the midbrain floor plate region, and the "absolute value of the rate of change in the NT-3 concentration" is measured when cells of interest that achieve a goal are obtained, i.e., when differentiation induction efficiency is favorable. Such culture and measurement are performed a plurality of times (at least twice, preferably at least five times), and an average value of the respectively measured "absolute values of the rates of change in the concentration of NT-3" can be set as the reference rate of change. Alternatively, pluripotent stem cells are cultured using a medium and culture conditions that allow for induction of differentiation, and an absolute value of the rate of change that offers sensitivity and specificity necessary for a user can be set as the reference rate of change on the basis of the relationship between the reference rate of change and sensitivity and specificity estimated from a ROC curve derived from the "absolute value of the rate of change in the concentration of NT-3" when cells of interest that achieve a goal are obtained, and the "absolute value of the rate of change in the concentration of NT-3" when the cells of interest that achieve a goal are not obtained, i.e., when differentiation induction efficiency is poor. In this context, the phrases "when cells of interest that achieve a goal are obtained" and "when differentiation induction efficiency is favorable" as well as the phrases "when the cells of interest that achieve a goal are not obtained" and "when differentiation induction efficiency is poor" are as defined in the section "Comparing measured concentration of NT-3 with reference concentration> for the first method.

In one aspect of the present embodiment, when the second culture supernatant is collected earlier than the first culture supernatant, the respective timings of collecting the first and second culture supernatants can be set such that the concentration of NT-3 in the first culture supernatant higher than the concentration of NT-3 in the second culture supernatant can be detected. In this case, the rate of change mentioned above may be calculated, or assessment may be made on the basis of the NT-3 concentration in the first culture supernatant higher than the NT-3 concentration in the second culture supernatant.

In one aspect of the present embodiment, when the second culture supernatant is collected later than the first culture supernatant, the respective timings of collecting the first and second culture supernatants can be set such that the concentration of NT-3 in the second culture supernatant lower than the concentration of NT-3 in the first culture supernatant can be detected. In this case, the rate of change mentioned above may be calculated, or assessment may be made on the basis of the NT-3 concentration in the second culture supernatant lower than the NT-3 concentration in the first culture supernatant.

The reference rate of change is preferably set to one point between 0.1 pg/ml-day or more and 500 pg/ml-day or less, and more preferably set to one point between 5 pg/ml-day or more and 50 pg/ml-day or less.

### <Assessing cells in culture broth as capable of differentiating into neural cells of midbrain floor plate region>

This step comprises, when the absolute value of the rate of change in the concentration of NT-3 is equal to or more than the reference rate of change, assessing the cells in the culture broth as capable of differentiating into the neural cells of the midbrain floor plate region.

In one aspect of the present embodiment, the phrase "the absolute value of the rate of change in the concentration of NT-3 is equal to or more than the reference rate of change" conceptually includes the case where the absolute value of the rate of change in the concentration of NT-3 is equivalent to the reference rate of change, and the case where the absolute value of the rate of change in the concentration of NT-3 is higher than the reference rate of change. For example, when the absolute value of the rate of change in the concentration of NT-3 is 1 to 10000 times the reference rate of change, the cells in the culture broth are preferably assessed as capable of differentiating into the neural cells of the midbrain floor plate region. When the absolute value of the rate of change in the concentration of NT-3 is 1 to 1000 times the reference rate of change, the cells in the culture broth are more preferably assessed as capable of differentiating into the neural cells of the midbrain floor plate region.

The ratio at which the cell population assessed as capable of differentiating into the neural cells of the midbrain floor plate region differentiates into the neural cells of the midbrain floor plate region may vary depending on culture conditions and the pluripotent stem cells used. The cells are considered to differentiate into the neural cells of the midbrain floor plate region at a ratio of, for example, 10% or more, 20% or more, 30% or more, 40% or more, 50% or more, 60% or more, 70% or more, 80% or more, or 90% or more, based on all the cells cultured.

In one aspect of the present embodiment, when the absolute value of the rate of change in the concentration of NT-3 is less than the reference rate of change, the cells in the culture broth or the culture broth itself may be discarded from the viewpoint of efficiently inducing differentiation. It is desirable to appropriately change the determination of whether to perform the discarding and the timing of the discarding according to a manufacturing method, a culture scale, the type and line of the cells used, a finally necessary amount of the cells, the targeted grade, the presence or absence of preculture and the status of progression thereof, the whole manufacturing schedule, the presence or absence of other determination methods, the number of days required for the determination, and cost, etc.

### <<Method for assessing differentiation potential of cells in culture broth in differentiation of pluripotent stem cells into neural cells of midbrain floor plate region - (3)>>

The third method for assessing differentiation potential of cells in a culture broth in the differentiation of pluripotent stem cells into neural cells of the midbrain floor plate region according to the present embodiment is
a method for assessing differentiation potential of cells in a culture broth in the differentiation of pluripotent stem cells into neural cells of the midbrain floor plate region, comprising:
measuring a concentration of NT-3 in each of a first culture supernatant, a second culture supernatant, and a third culture supernatant in a culture broth obtained by culturing the pluripotent stem cells in a medium containing an inducer of differentiation into the neural cells of the midbrain floor plate region;
comparing the concentration of NT-3 in the first culture supernatant, the concentration of NT-3 in the second culture supernatant, and the concentration of NT-3 in the third culture supernatant; and
when the concentration of NT-3 in the first culture supernatant is higher than the concentration of NT-3 in the second culture supernatant and the concentration of NT-3 in the third culture supernatant, assessing the cells in the culture broth as capable of differentiating into the neural cells of the midbrain floor plate region, wherein
the first culture supernatant is a culture supernatant collected from the culture broth at any time from 48 hours to 192 hours after the start of culture of the pluripotent stem cells,
the second culture supernatant is a culture supernatant collected from the culture broth at any time from 24 hours to 96 hours before collection of the first culture supernatant, and
the third culture supernatant is a culture supernatant collected from the culture broth at any time from 24 hours to 96 hours after collection of the first culture supernatant.

### <Measuring concentration of NT-3>

This step comprises measuring a concentration of NT-3 in each of a first culture supernatant, a second culture supernatant, and a third culture supernatant in a culture broth obtained by culturing the pluripotent stem cells in a medium containing an inducer of differentiation into the neural cells of the midbrain floor plate region. The method for measuring the concentration can adopt the measurement method described in the first method mentioned above.

The first culture supernatant may be a culture supernatant collected from the culture broth at any time from 48 hours to 192 hours after the start of culture of the pluripotent stem cells, or may be a culture supernatant collected from the culture broth at any time from 48 hours to 96 hours after the start of culture of the pluripotent stem cells. The first culture supernatant is more preferably a culture supernatant collected from the culture broth at any time from 48 hours to 72 hours after the start of culture of the pluripotent stem cells.

In one aspect, the second culture supernatant is collected from the culture broth at the start of differentiation or at a later timing and before collection of the first culture supernatant, for example, 24 hours to 96 hours (preferably 24 hours to 72 hours) after the start of differentiation and before collection of the first culture supernatant. Specifically, the second culture supernatant is a culture supernatant collected from the culture broth at any time from 24 hours to 96 hours (more preferably 24 hours to 72 hours) before collection of the first culture supernatant, and is preferably a culture supernatant collected from the culture broth any time from 24 hours to 48 hours before collection of the first culture supernatant.

In this context, in one aspect of the present embodiment, the respective timings of collecting the first and second culture supernatants can be set such that the concentration of NT-3 in the second culture supernatant higher than the concentration of NT-3 in the first culture supernatant can be detected.

The third culture supernatant is a culture supernatant collected from the culture broth at any time from 24 hours or later after collection of the first culture supernatant to the completion of differentiation into the neural cells of the midbrain floor plate region, and is preferably a culture supernatant collected from the culture broth at any time from 24 hours to 96 hours after collection of the first culture supernatant, more preferably a culture supernatant collected from the culture broth at any time from 24 hours to 72 hours after collection of the first culture supernatant.

In one aspect of the present embodiment, the respective timings of collecting the first and third culture supernatants can be set such that the concentration of NT-3 in the third culture supernatant lower than the concentration of NT-3 in the first culture supernatant can be detected.

In this context, the "completion of differentiation into the neural cells of the midbrain floor plate region" means a state where cell differentiation progresses to the extent that a marker of the neural cells of the midbrain floor plate region is expressed. In this respect, neural cells into which the neural cells of the midbrain floor plate region further differentiate may be contained in the culture broth. Specific examples thereof include a differentiation stage at which a plurality of markers of the neural cells of the midbrain floor plate region are detected. Examples of the markers can include Corin, Lrtm1, Foxa2, Lmx1a, Lmx1b, and CD142.

The composition of the medium for culturing the pluripotent stem cells can adopt the composition described in the first method mentioned above.

### <Comparing concentration of NT-3>

This step comprises comparing the concentration of NT-3 in the first culture supernatant, the concentration of NT-3 in the second culture supernatant, and the concentration of NT-3 in the third culture supernatant.

### <Assessing cells in culture broth as capable of differentiating into neural cells of midbrain floor plate region>

This step comprises, when the concentration of NT-3 in the first culture supernatant is higher than the concentration of NT-3 in the second culture supernatant and the concentration of NT-3 in the third culture supernatant, assessing the cells in the culture broth as capable of differentiating into the neural cells of the midbrain floor plate region.

In one aspect of the present embodiment, when the difference between the concentration of NT-3 in the first culture supernatant and a lower one of the respective concentrations of NT-3 in the second culture supernatant and the third culture supernatant is 5 pg/ml or more, the cells in the culture broth are preferably assessed as capable of differentiating into the neural cells of the midbrain floor plate region. When the difference between the concentrations is 10 pg/ml or more, the cells in the culture broth are more preferably assessed as capable of differentiating into the neural cells of the midbrain floor plate region.

The ratio at which the cell population assessed as capable of differentiating into the neural cells of the midbrain floor plate region differentiates into the neural cells of the midbrain floor plate region may vary depending on culture conditions and the pluripotent stem cells used. The cells are considered to differentiate into the neural cells of the midbrain floor plate region at a ratio of, for example, 10% or more, 20% or more, 30% or more, 40% or more, 50% or more, 60% or more, 70% or more, 80% or more, or 90% or more, based on all the cells cultured.

In one aspect of the present embodiment, when the concentration of NT-3 in the first culture supernatant is the same as or lower than the concentration of NT-3 in the second culture supernatant and the concentration of NT-3 in the third culture supernatant, the cells in the culture broth or the culture broth itself may be discarded from the viewpoint of efficiently inducing differentiation. It is desirable to appropriately change the determination of whether to perform the discarding and the timing of the discarding according to a manufacturing method, a culture scale, the type and line of the cells used, a finally necessary amount of the cells, the targeted grade, the presence or absence of preculture and the status of progression thereof, the whole manufacturing schedule, the presence or absence of other determination methods, the number of days required for the determination, and cost, etc.

### <Assessing method using plurality of factors>

In one embodiment, the assessing method of the present invention may be carried out by combining the NT-3 mentioned above with one or more substances, other than the NT-3, which are detected in the culture supernatant in the process of differentiation into neural cells of the midbrain floor plate region, as substances serving as an index for assessment.

The "one or more substances other than the NT-3" are not particularly limited and can be appropriately selected as a substance determined to have usefulness for the assessing method of the present invention, for example, by univariate logistic regression analysis, among substances whose concentration in the culture supernatant is found to correlate with differentiation into neural cells of the midbrain floor plate region.

In the case of performing the assessing method of the present invention by combining the NT-3 and "one or more substances other than the NT-3 ", the optimum model formula for assessing differentiation potential into neural cells of the midbrain floor plate region in the combination is determined in advance by, for example, multivariate logistic regression analysis or Cox regression analysis, and the differentiation potential into neural cells of the midbrain floor plate region may be assessed on the basis of the model formula.

### <Assessing method using culture apparatus>

In one aspect of the present embodiment, the assessing method can be used in a culture apparatus for inducing the differentiation of pluripotent stem cells. Examples of the culture apparatus include a culture apparatus having a culture tank which cultures cells of interest in a liquid medium, a medium supply unit which supplies a fresh liquid medium to the culture tank, and a medium discharge unit which discharges the liquid medium from the culture tank. The liquid medium discharged for the replacement of a collected medium by the medium discharge unit corresponds to the culture supernatant in the aforementioned methods (1) to (3) for assessing differentiation potential of cells in a culture broth in the differentiation of pluripotent stem cells into neural cells of the midbrain floor plate region. Examples of the culture apparatus include a large-scale automated iPS cell culture apparatus "iACE2" manufactured by Hitachi, Ltd.

The culture apparatus may have a measurement unit which measures the concentration of NT-3 contained in the discharged liquid medium, and a differentiation potential assessment unit which assesses whether the cells of interest are capable of differentiating into the neural cells of the midbrain floor plate region on the basis of the measured concentration of NT-3, in addition to the culture tank which cultures cells of interest in a liquid medium, the medium supply unit which supplies a fresh liquid medium to the culture tank, and the medium discharge unit which discharges the liquid medium from the culture tank.

The culture apparatus has a system for automatically performing medium replacement during cell culture and can discharge a medium and inject a fresh medium. Hence, the discharged medium, i.e., a liquid waste, can be noninvasively collected and subjected to component analysis to carry out the assessing method of the present invention. For example, an analyzer (measurement unit) for measuring NT-3 contained in the collected liquid waste at the time of medium replacement is integrated in the culture apparatus, and a lot having differentiation potential into neural cells of the midbrain floor plate region may be selected and continuously cultured.

In the culture apparatus, a completely closed flow channel module is preferably used in a culture vessel and medium flow channels from the viewpoint of performing cell inoculation, culture, and observation in a sterile environment.

The method for assessing differentiation potential of cells in a culture broth in the differentiation of pluripotent stem cells into neural cells of the midbrain floor plate region according to the present embodiment is described above. The assessing method is useful in an established method for manufacturing dopaminergic progenitor cells serving as an active ingredient for a cell medicament, which are obtained by inducing the differentiation of pluripotent stem cells. In the case of carrying out the assessing method, it is desirable to carry out in advance the method for manufacturing dopaminergic progenitor cells to determine the secretion pattern of NT-3, and then determine the optimum timing of measuring the amount of NT-3 secreted in an individual manufacturing method. The assessing method is preferably applicable to a manufacturing method capable of manufacturing more than a certain level of Corin-positive cells as a method for manufacturing at least cells of the midbrain floor plate region (also called midbrain floor plate cells), i.e., Corin-positive cells.

The assessing method is preferably used in inducing the differentiation of iPS cells cultured for the maintenance of an undifferentiated state by a method well known to those skilled in the art. Specifically, the assessing method can be preferably adapted to a culture method for maintaining the pluripotency of iPS cells as long as usable iPS cells cultured under proper culture conditions are used.

### <<Method for manufacturing dopaminergic progenitor cells or precursor cells thereof>>

The method for manufacturing dopaminergic progenitor cells or precursor cells thereof according to the present embodiment is
a method for manufacturing dopaminergic progenitor cells or precursor cells thereof from pluripotent stem cells, comprising:
(A) starting the culture of the pluripotent stem cells under culture conditions capable of inducing differentiation into neural cells of the midbrain floor plate region;
(B) performing the method for assessing differentiation potential of cells in a culture broth in the differentiation of pluripotent stem cells into neural cells of the midbrain floor plate region between 0 hours and 288 hours after the start of culture of the pluripotent stem cells;
(C) determining to further continue the culture of the cells in the culture broth assessed as capable of differentiating into the neural cells of the midbrain floor plate region in the step (B), and allowing the cells in the culture broth to differentiate into the neural cells of the midbrain floor plate region; and
(D) culturing the neural cells of the midbrain floor plate region under culture conditions capable of inducing differentiation into the dopaminergic progenitor cells so that the cells are allowed to differentiate into the dopaminergic progenitor cells or precursor cells thereof.

### <Step (A) of starting culture of pluripotent stem cells>

The step (A) comprises starting the culture of the pluripotent stem cells under culture conditions capable of inducing differentiation into neural cells of the midbrain floor plate region. The cells mentioned above may be used as the pluripotent stem cells.

### (Culture conditions capable of inducing differentiation into neural cells of midbrain floor plate region)

The culture conditions (medium, temperature, carbon dioxide concentration, etc.) capable of inducing differentiation into neural cells of the midbrain floor plate region will be described below. The composition of the medium for culturing the pluripotent stem cells can adopt the composition described in the first method mentioned above.

The temperature at which the pluripotent stem cells are cultured is preferably 30°C or more and 40°C or less, more preferably 36°C or more and 38°C or less.

The carbon dioxide concentration at which the pluripotent stem cells are cultured is preferably 2% or more and 5% or less, more preferably 4% or more and 5% or less.

The cell density at which the pluripotent stem cells are cultured is preferably 10 cells/cm² or more and 10000000 cells/cm² or less, more preferably 100 cells/cm² or more and 1000000 cells/cm² or less.

In one aspect of the present embodiment, the step (A) preferably comprises adhesion-culturing the pluripotent stem cells on extracellular matrix. The phrase "adhesion-culturing on extracellular matrix" means culturing using a culture vessel coated with the extracellular matrix. The coating treatment may be performed by placing a solution containing the extracellular matrix in a culture vessel and then appropriately removing the solution.

### <Step (B) of performing method for assessing differentiation potential of cells in culture broth in differentiation of pluripotent stem cells into neural cells of midbrain floor plate region>

The step (B) comprises performing the method for assessing differentiation potential of cells in a culture broth in the differentiation of pluripotent stem cells into neural cells of the midbrain floor plate region between 0 hours and 288 hours after the start of culture of the pluripotent stem cells. In one aspect of the present embodiment, the step (B) may be interpreted as completing assessment by the method for assessing differentiation potential of cells in a culture broth in the differentiation of pluripotent stem cells into neural cells of the midbrain floor plate region between 0 hours and 288 hours after the start of culture of the pluripotent stem cells. In one aspect of the present embodiment, the phrase "between 0 hours and 288 hours after the start of culture of the pluripotent stem cells" may be interpreted as "up to the production of neural cells of the midbrain floor plate region from the pluripotent stem cells" and may be interpreted as "up to the production of at least 10%, 20%, 30%, 40%, 50%, 60%, 70%, 80% or 90% or more (based on the total number of cells) of neural cells of the midbrain floor plate region from the pluripotent stem cells".

In the step (B), any of the first method, the second method, and the third method mentioned above may be used. The cells (e.g., pluripotent stem cells and cells derived from the pluripotent stem cells) in the culture broth are also continuously cultured while the step (B) is performed.

### <Step (C) of allowing cells in culture broth to differentiate into neural cells of midbrain floor plate region>

The step (C) comprises determining to further continue the culture of the cells in the culture broth assessed as capable of differentiating into the neural cells of the midbrain floor plate region in the step (B), and allowing the cells in the culture broth to differentiate into the neural cells of the midbrain floor plate region. The cells (e.g., pluripotent stem cells and cells derived from the pluripotent stem cells) in the culture broth are also continuously cultured while the step (C) is performed.

The culture period for which the cells (e.g., pluripotent stem cells and cells derived from the pluripotent stem cells) in the culture broth are cultured is preferably 9 days or more and 21 days or less, more preferably 9 days or more and 16 days or less, in terms of the period from the step (A) to the step (C).

In one aspect of the present embodiment, the culture of the cells for the period from the step (A) to the step (C) may be performed by multiple stages using one or more BMP signaling-inhibiting substances, one or more TGFβ signaling-inhibiting substances, one or more SHH signaling-activating substances, and one or more Wnt signaling-activating substances as the inducer of differentiation. At each of the stages in the culture of the cells, the culture may be performed in a medium containing at least one substance selected from the group consisting of a BMP signaling-inhibiting substance, a TGFβ signaling-inhibiting substance, a SHH signaling-activating substance, and a Wnt signaling-activating substance, and may be adhesion culture or suspension culture. The step (B) is desirably performed between the multiple stages.

In one aspect of the present embodiment, the inducer of differentiation for use in the culture of the cells for the period from the step (A) to the step (C) desirably further comprises FGF8. Specifically, the culture of the cells for the period from the step (A) to the step (C) may be performed by multiple stages using one or more BMP signaling-inhibiting substances, one or more TGFβ signaling-inhibiting substances, one or more SHH signaling-activating substances, one or more Wnt signaling-activating substances, and FGF8 as the inducer of differentiation. At each of the stages in the culture of the cells, the culture may be performed in a medium containing at least one substance selected from a BMP signaling-inhibiting substance, a TGFβ signaling-inhibiting substance, a SHH signaling-activating substance, a Wnt signaling-activating substance, and FGF8, and may be adhesion culture or suspension culture. The step (B) is desirably performed between the multiple stages.

In one aspect of the present embodiment, the culture of the cells for the period from the step (A) to the step (C) may be performed by multiple stages using one or more BMP signaling-inhibiting substances, one or more TGFβ signaling-inhibiting substances, one or more SHH signaling-activating substances, and one or more Wnt signaling-activating substances, as the inducer of differentiation. In one aspect, one or more SHH signaling-activating substances and one or more Wnt signaling-activating substances may be used at the same time with the timing of using one or more BMP signaling-inhibiting substances and one or more TGFβ signaling-inhibiting substances. Alternatively, one or more SHH signaling-activating substances and one or more Wnt signaling-activating substances may be used after a lapse of several days from the start of culture in a culture broth containing BMP signaling-inhibiting substances and TGFβ signaling-inhibiting substances. The SHH signaling-activating substances and the Wnt signaling-activating substances may be used in a culture broth at different timings or at the same time.

In one aspect of the present embodiment, in the step (A) to the step (C), it is desirable to culture the pluripotent stem cells by the following multiple stages and perform the step (B) during the culture:
(a) adhesion-culturing the pluripotent stem cells on extracellular matrix in a culture broth containing a BMP signaling-inhibiting substance and a TGFβ signaling-inhibiting substance;
(b) adhesion-culturing the cells obtained in the step (a) on extracellular matrix in a culture broth containing a BMP signaling-inhibiting substance, a TGFβ signaling-inhibiting substance, a SHH signaling-activating substance, and FGF8;
(c) adhesion-culturing the cells obtained in the step (b) on extracellular matrix in a culture broth containing a BMP signaling-inhibiting substance, a TGFβ signaling-inhibiting substance, a SHH signaling-activating substance, FGF8, and a Wnt signaling-activating substance; and
(d) adhesion-culturing the cells obtained in the step (c) on extracellular matrix in a culture broth containing a BMP signaling-inhibiting substance and a Wnt signaling-activating substance.

In one aspect of the present embodiment, the step (B) is preferably performed during the period from the step (a) to the step (c). In another aspect of the present embodiment, the protocol of the culture related to the step (A) to the step (C) may adopt a protocol known in the art (e.g., Frontiers in Cell and Developmental Biology, August 2020, Volume 8, Article 729; and Cell Stem Cell 28, 343-355, February 4, 2021).

In the present embodiment, the extracellular matrix is an extracellularly existing supramolecular structure and may be naturally derived or may be an artificial product (recombinant). Examples thereof include substances such as collagen, proteoglycan, fibronectin, hyaluronic acid, tenascin, entactin, elastin, fibrillin, and laminin, and their fragments. These extracellular matrices may be used in combination and may be preparations from cells, for example, BD Matrigel(TM). The extracellular matrix is preferably laminin or a fragment thereof. In the present embodiment, the laminin is a protein having a heterotrimer structure having one each of α, β, and γ chains. Examples thereof include, but are not particularly limited to, α1, α2, α3, α4, and α5 as the α chain, β1, β2, and β3 as the β chain, and γ1, γ2, and γ3 as the γ chain. The laminin is more preferably laminin 511 composed of α5, β1, and γ1. In the present embodiment, the laminin may be a fragment. The laminin fragment is not particularly limited as long as the fragment has integrin-binding activity. The laminin fragment may be, for example, an E8 fragment which is a fragment obtained by digestion with elastase. Thus, in the present embodiment, examples thereof include laminin 511E8 (preferably human laminin 511E8) described in WO2011/043405.

Examples of the number of days for which the step (a) is performed include 1 day or more, 2 days or more, 3 days or more, 4 days or more, 5 days or more, 6 days or more, 7 days or more, and larger numbers of days. The number of days for which the step (a) is performed is preferably 1 day. Likewise, examples of the number of days for which the step (b) is performed include 1 day or more, 2 days or more, 3 days or more, 4 days or more, 5 days or more, 6 days or more, 7 days or more, and larger numbers of days. The number of days for which the step (b) is performed is preferably 2 days. Likewise, examples of the number of days for which the step (c) is performed include 1 day or more, 2 days or more, 3 days or more, 4 days or more, 5 days or more, 6 days or more, 7 days or more, and larger numbers of days. The number of days for which the step (c) is performed is preferably 4 days. Likewise, examples of the number of days for which the step (d) is performed include 1 day or more, 2 days or more, 3 days or more, 4 days or more, 5 days or more, 6 days or more, 7 days or more, and larger numbers of days. The number of days for which the step (d) is performed is preferably 5 days or more.

The pluripotent stem cells may be used in the form of dissociated cells. Examples of the method for dissociating the cells include a dynamic dissociation method and a dissociation method using dissociation solutions having protease activity and collagenase activity (e.g., Accutase(TM) and Accumax(TM)) or a dissolution solution having only collagenase activity. A method of dissociating human pluripotent stem cells using trypsin or a replacement thereof (examples thereof include TrypLE CTS (Life Technologies)) is preferably used. The dissociated cells are desirably cultured by appropriately adding a ROCK inhibitor after dissociation. In the case of adding a ROCK inhibitor, the cells can be cultured for at least 1 day, more preferably 1 day, by the addition.

### <ROCK inhibitor>

In the present embodiment, the ROCK inhibitor is not particularly limited as long as the ROCK inhibitor can suppress the function of Rho kinase (ROCK). Examples thereof include Y-27632 (e.g., see Ishizaki et al., Mol. Pharmacol. 57, 976-983 (2000); and Narumiya et al., Methods Enzymol. 325, 273-284 (2000)), Fasudil/HA1077 (e.g., see Uenata et al., Nature 389: 990-994 (1997)), H-1152 (e.g., see Sasaki et al., Pharmacol. Ther. 93: 225-232 (2002)), Wf-536 (e.g., see Nakajima et al., Cancer Chemother Pharmacol. 52 (4): 319-324 (2003)), and derivatives thereof, and antisense nucleic acids, RNA interference-inducing nucleic acids (e.g., siRNA), and dominant negative mutants against ROCK, and expression vectors thereof. Other low-molecular compounds are also known as the ROCK inhibitor. In the present embodiment, such a compound or a derivative thereof may be used (e.g., see U.S. Patent Application Publication Nos. 20050209261, 20050192304, 20040014755, 20040002508, 20040002507, 20030125344, and 20030087919, and WO2003/062227, WO2003/059913, WO2003/062225, WO2002/076976, and WO2004/039796). In the present embodiment, one or two or more ROCK inhibitors may be used. The ROCK inhibitor used in the present embodiment can be preferably Y-27632.

The concentration of Y-27632 in the culture broth is, for example, 100 nM to 50 µM, preferably 1 µM to 10 µM, more specifically 100 nM, 500 nM, 750 nM, 1 µM, 2 µM, 3 µM, 4 µM, 5 µM, 6 µM, 7 µM, 8 µM, 9 µM, 10 µM, 15 µM, 20 µM, 25 µM, 30 µM, 40 µM, or 50 µM, though the concentration is not limited thereto.

### <Step (D) of allowing neural cells of midbrain floor plate region to differentiate into dopaminergic progenitor cells or precursor cells thereof>

The step (D) comprises culturing the neural cells of the midbrain floor plate region under culture conditions capable of inducing differentiation into the dopaminergic progenitor cells so that the cells are allowed to differentiate into the dopaminergic progenitor cells or precursor cells thereof. In one aspect of the present embodiment, the step (D) may be interpreted as changing the culture conditions capable of inducing differentiation into the neural cells of the midbrain floor plate region to culture conditions capable of inducing differentiation into the dopaminergic progenitor cells, and culturing the neural cells of the midbrain floor plate region so that the cells differentiate into the dopaminergic progenitor cells or the precursor cells thereof.

The culture conditions (medium, temperature, carbon dioxide concentration, etc.) capable of inducing differentiation into the dopaminergic progenitor cells will be described below.

The medium for culturing the neural cells of the midbrain floor plate region can be prepared with a medium for use in animal cell culture as a basal medium. The basal medium encompasses, for example, Glasgow's Minimal Essential Medium (GMEM), IMDM medium, Medium 199, Eagle's Minimum Essential Medium (EMEM), aMEM medium, Dulbecco's modified Eagle's Medium (DMEM), Ham's F12 medium, RPMI1640 medium, Fischer's medium, Neurobasal Medium (Life Technologies Corp.), and mixed media thereof. Neurobasal Medium is preferred. The medium may contain serum or may be serum-free. The medium may optionally contain, for example, one or more serum replacements such as albumin, transferrin, Knockout Serum Replacement (KSR) (serum replacement of FBS for ES cell culture), N2 supplement (Invitrogen), B27 supplement (Invitrogen), fatty acid, insulin, procollagen, trace element, 2-mercaptoethanol, and 3'-thiolglycerol, and may contain one or more substances such as a lipid, an amino acid, L-glutamine, Glutamax (Invitrogen), nonessential amino acids, a vitamin, a growth factor, a low-molecular compound, an antibiotic, an antioxidant, pyruvic acid, a buffer, inorganic salts, and a nucleic acid (e.g., Dibutyryl cyclic AMP (dbcAMP)). The culture broth is preferably Neurobasal Medium containing B27 supplement, ascorbic acid, and dbcAMP. This culture broth is appropriately supplemented with a neurotrophic factor for culture.

### <Neurotrophic factor>

In the present embodiment, the neurotrophic factor is a ligand for a membrane receptor that plays an important role in the survival and functional maintenance of neurons. Examples thereof include Nerve Growth Factor (NGF), Brain-derived Neurotrophic Factor (BDNF), Neurotrophin-3 (NT-3), Neurotrophin-4/5 (NT-4/5), Neurotrophin-6 (NT-6), basic FGF, acidic FGF, FGF-5, Epidermal Growth Factor (EGF), Hepatocyte Growth Factor (HGF), Insulin, Insulin Like Growth Factor 1 (IGF1), Insulin Like Growth Factor 2 (IGF2), Glia cell line-derived Neurotrophic Factor (GDNF), TGF-b2, TGF-b3, Interleukin 6 (IL-6), Ciliary Neurotrophic Factor (CNTF), and LIF.

Examples of the neurotrophic factor preferably include Nerve Growth Factor (NGF), Brain-derived Neurotrophic Factor (BDNF), Neurotrophin-3 (NT-3), Neurotrophin-4/5 (NT-4/5), Neurotrophin-6 (NT-6), Glia cell line-derived Neurotrophic Factor (GDNF), TGF-b3, and Ciliary Neurotrophic Factor (CNTF).

The neurotrophic factor is preferably selected from the group consisting of GDNF, BDNF, and NT-3. GDNF or BDNF, preferably GDNF and BDNF, are preferably used as the neurotrophic factor. The neurotrophic factor is commercially available from, for example, FUJIFILM Wako Pure Chemical Corp. or R&D systems, Inc. and may be readily utilized. The neurotrophic factor may be obtained by forced expression in cells by a method known to those skilled in the art.

The concentration of GDNF in the culture broth is, for example, 0.1 ng/mL, 0.5 ng/mL, 1 ng/mL, 5 ng/mL, 10 ng/mL, 15 ng/mL, 20 ng/mL, 25 ng/mL, 30 ng/mL, 40 ng/mL, 50 ng/mL, 100 ng/mL, 200 ng/mL, or 500 ng/mL, though the concentration is not limited thereto. The concentration is preferably 10 ng/mL. In one aspect of the present embodiment, the concentration of GDNF in the culture broth may be 0.1 ng/mL or more and 500 ng/mL or less or may be 1 ng/mL or more and 100 ng/mL or less.

The concentration of BDNF in the culture broth is, for example, 0.1 ng/mL, 0.5 ng/mL, 1 ng/mL, 5 ng/mL, 10 ng/mL, 15 ng/mL, 20 ng/mL, 25 ng/mL, 30 ng/mL, 40 ng/mL, 50 ng/mL, 100 ng/mL, 200 ng/mL, or 500 ng/mL, though the concentration is not limited thereto. The concentration is preferably 20 ng/mL. The concentration of BDNF in the culture broth may be 0.1 ng/mL or more and 500 ng/mL or less or may be 1 ng/mL or more and 100 ng/mL or less.

The temperature at which the neural cells of the midbrain floor plate region are cultured is preferably 30°C or more and 40°C or less, more preferably 35°C or more and 38°C or less.

The carbon dioxide concentration at which the neural cells of the midbrain floor plate region are cultured is preferably 2% or more and 5% or less, more preferably 4% or more and 5% or less.

The cell density at which the neural cells of the midbrain floor plate region are cultured is preferably 50 cells/ml or more and 100000 cells/ml or less, more preferably 500 cells/ml or more and 50000 cells/ml or less.

The culture period for which the neural cells of the midbrain floor plate region are cultured is preferably 7 days or more and 30 days or less, more preferably 7 days or more and 24 days or less, in terms of the period of the step (D).

The step (D) may be performed by suspension culture. The suspension culture is culture in the state of cells nonadherent to a culture vessel and is not particularly limited. The suspension culture can be performed using a culture vessel that has not undergone artificial treatment (e.g., coating treatment with extracellular matrix) for the purpose of improving adhesion to cells, or a culture vessel that has undergone artificial treatment to suppress adhesion (e.g., with poly(hydroxyethyl methacrylate) (poly-HEMA), a nonionic surfactant polyol (Pluronic F-127, etc.), or a phospholipid-like structure (e.g., a water-soluble polymer (Lipidure) having 2-methacryloyloxyethyl phosphorylcholine as a constituent unit).

In one aspect of the present embodiment, the step (D) is preferably performed by selecting and collecting midbrain floor plate cells from the neural cells of the midbrain floor plate region, and culturing the collected midbrain floor plate cells under culture conditions capable of inducing differentiation into the dopaminergic progenitor cells.

The method for selecting and collecting midbrain floor plate cells from the neural cells of the midbrain floor plate region can be performed using a substance that specifically binds to a marker molecule present on the cell surface of the midbrain floor plate cells in order to select the midbrain floor plate cells from the cell population. The substance that binds to a marker molecule can adopt an antibody or an aptamer and is preferably an antibody or an antigen-binding fragment thereof.

In the present embodiment, the antibody can be a polyclonal or monoclonal antibody. Such an antibody may be prepared by a use of a technique well known to those skilled in the art (Current protocols in Molecular Biology edit. Ausubel et al.

(1987) Publish. John Wiley and Sons. Section 11.12-11.13). The monoclonal antibody can be obtained from hybridoma cells prepared by the cell fusion between splenocytes obtained from a nonhuman animal immunized with the marker molecule and myeloma cells (Current protocols in Molecular Biology edit. Ausubel et al. (1987) Publish. John Wiley and Sons. Section 11.4-11.11). Examples of the antigen-binding fragment of the antibody include a portion (e.g., a Fab fragment) of the antibody and synthetic antibody fragments (e.g., single-chain Fv fragments "ScFv"). Antibody fragments such as Fab and F(ab)₂ fragments can also be prepared by a well-known genetic engineering method.

The substance that binds to the marker molecule may be bound or conjugated with, for example, a detectable substance such as a fluorescent label, a radiolabel, a chemiluminescent label, an enzyme, biotin, or streptavidin, or a substance that allows for isolation or extraction such as protein A, protein G, beads, or magnetic beads, for the purpose of recognizing or separating the midbrain floor plate cells expressing the marker molecule. The substance that binds to the marker molecule may be indirectly labeled. Such labeling may be performed by use of various methods known to those skilled in the art. Examples thereof include a method using a preliminarily labeled antibody (secondary antibody) that specifically binds to the antibody.

The method for detecting cells of interest such as midbrain floor plate cells involves using a flow cytometer or a protein chip, etc.

Examples of the method for extracting cells of interest such as midbrain floor plate cells include a method of conjugating particles to the substance that binds to the marker molecule to precipitate the particles, a method of selecting the cells through magneticity using magnetic beads (e.g., MACS), a method using a fluorescent label and using a cell sorter, and a method using an antibody-immobilized carrier (e.g., a cell enrichment column).

In the present embodiment, examples of the marker molecule for midbrain floor plate cells that may be used for selecting and collecting the cells of interest include CORIN, LRTM1, and CD142. The midbrain floor plate cells are conceptually encompassed by the neural cells of the midbrain floor plate region.

In one aspect of the present embodiment, the method preferably further comprises, between the step (C) and the step (D), (E) collecting neural cells of the midbrain floor plate region.

In this context, the collection means collecting the cells obtained in the step (C). For example, when adhesion culture has been performed or when a cell aggregate has been formed in suspension culture, the collection also includes operation of detaching the cells from a culture vessel or dispersing the aggregated cells, for example, by mechanical dispersion treatment (examples thereof include pipetting treatment and scraping treatment with a scraper) or cell-dispersing liquid treatment (examples thereof include treatment with an enzyme such as trypsin, collagenase, or papain, or a chelating agent such as ethylenediaminetetraacetic acid).

Using the cells collected in the step (E), the midbrain floor plate cells of interest are selected from the population of neural cells of the midbrain floor plate region, as mentioned above, and the cell population can be purified.

In one aspect, the selection of midbrain floor plate cells in the step (D) can be performed using a substance that specifically binds to Corin in order to select neural cells of the midbrain floor plate region from a cell population. A specific approach may employ a method similar to the aforementioned method for collecting the midbrain floor plate cells.

The dopaminergic progenitor cells and/or the precursor cells thereof (hereinafter, also referred to as "dopaminergic progenitor cells, etc.") manufactured by the method for manufacturing dopaminergic progenitor cells or precursor cells thereof according to the present embodiment may be used as a tissue for transplantation. Examples of the tissue for transplantation include tissues for transplantation to be transplanted into the brains of Parkinson's disease patients. The number of the dopaminergic progenitor cells, etc. in the tissue for transplantation is not particularly limited and is, for example, 5 × 10⁵ or more and 3 × 10⁷ or less cells to be transplanted. The shape of the tissue for transplantation may be sheeted, may be spherical, or may be a shape adapted to a transplantation site. The tissue for transplantation may be in the state of a suspension.

The HLA genotype of the cells contained in the tissue for transplantation is preferably the same or substantially the same as that of a recipient individual from the viewpoint that rejection does not occur.

The tissue for transplantation containing the dopaminergic progenitor cells, etc. can be transplanted to a diseased site by, for example, an approach as described in Nature Communication, 11, 3369 (2020), Nature Neuroscience, 2, 1137 (1999), or N Engl J Med.; 344: 710-9 (2001).

### Examples

Hereinafter, the present invention will be described with reference to Examples. However, the present invention is not limited by these examples.

### <<Experiment 1>>

### «Culture for maintenance of undifferentiated state»

Prior to culture for the induction of differentiation mentioned later, iPS cells were cultured while their undifferentiated state was maintained. Specifically, two types of culture conditions of the final stage of an undifferentiated state maintenance step were set under conditions of culture experiments A, B, and C and comparatively studied.

### <Culture experiment A>

An iPS cell line (formal name: 1231A3) as a line for research was used and cultured in a medium having composition given below. The medium was supplemented (with pretreatment) with SAG (N-Methyl-N'-(3-pyridinylbenzyl)-N'-(3-chlorobenzo[b]thiophene-2-carbonyl)-1,4-diaminocyclohexane, final concentration: 300 nM) for 1 day from 1 day before the start of differentiation culture (Day -1) to the start day of differentiation (Day 0) to establish a cell group having high induction efficiency (cell group A1). An untreated cell group (cell group A2) was also established for comparison by cell culture under the same conditions as in the cell group A1 except that the medium was not supplemented with SAG.

As for the medium in the culture experiment A, AK03N basal medium manufactured by Ajinomoto Co., Inc. was used before the start of differentiation, and GMEM containing 8% KSR was used as a basal medium at and after the start of differentiation (Day 0).

### <Culture experiment B>

An iPS cell line (QHJI01 s04 line) as a line for clinical research was used and cultured under similar culture conditions as in the cell group A2 in the culture experiment A. In the culture experiment B, cell groups B1 and B2 differing in cell culture period were established. The cell group B1 was a cell group having low differentiation induction efficiency, and the cell group B2 was a cell group having high differentiation induction efficiency.

### <Culture experiment C>

An iPS cell line (formal name: 201B7) as a line for research was used and cultured under similar culture conditions as in the cell group A2 in the culture experiment A. In the culture experiment C, cell groups C1 and C2 differing in medium component were established. The cell group C1 was a cell group having low differentiation induction efficiency, and the cell group C2 was a cell group having high differentiation induction efficiency.

### «Culture for induction of differentiation into neural cells of midbrain floor plate region»

The pluripotent stem cells in each of six types of cell groups provided in the culture experiments A, B, and C were induced to differentiate into neural cells of the midbrain floor plate region in accordance with a predetermined protocol. Specifically, the addition of A-83-01 (TGFβ signaling-inhibiting substance, one kind of SMAD signaling-inhibiting substance, final concentration: 0.5 µM) and LDN-193189 (BMP signaling-inhibiting substance, one kind of SMAD signaling-inhibiting substance, final concentration: 0.1 µM) to the medium was started on 0 days from the start of differentiation. The addition of Purmorphamine (SHH signaling-activating substance, final concentration: 2 µM) and FGF8 (final concentration: 100 ng/mL) to the medium was started 1 day after the start of differentiation. The addition of CHIR99021 (Wnt signaling-activating substance, final concentration: 3 µM) was started 3 days after the start of differentiation. The addition of A-83-01, Purmorphamine, and FGF8 was terminated 7 days after the start of differentiation. The whole amount of the medium was replaced every 24 hours from the start of differentiation through 13 days later. If the composition of the medium was changed in accordance with the protocol, the culture was shifted to the one in a medium having different composition at the timing of medium replacement. The ratio of Corin-positive cells in each cell group was measured using a cell sorter (BD FACSJazz, BD Accuri or Furukawa PERFLOW Sort) on 12 days from the start of differentiation. The manufacturer of each reagent is as follows.
A-83-01: manufactured by FUJIFILM Wako Pure Chemical Corp.
LDN-193189: manufactured by Stemgent
Purmorphamine: manufactured by FUJIFILM Wako Pure Chemical Corp.
FGF8: manufactured by FUJIFII,M Wako Pure Chemical Corp.
CHIR99021: manufactured by FUJIFILM Wako Pure Chemical Corp.

### <Concentration analysis of NT-3>

A portion of the culture supernatant in each cell group was collected every 24 hours or every 48 hours from immediately after the start of culture for the induction of differentiation (i.e., immediately after the start of differentiation), and analyzed for the concentration of NT-3. The timing of collecting the culture supernatant was set to a fixed time of 24 hours (1 day) according to the timing of medium replacement described above. In collecting the culture supernatant, the whole amount of the medium was temporarily collected into a container different from the culture vessel, and a portion thereof was collected in a well stirred state as a whole. The results are shown in Figs. 1 to 3. The concentration of NT-3 was analyzed using Bioplex(R) manufactured by Bio-Rad Laboratories, Inc.

### «Results»

### <Culture experiment A>

The cell group A1 had a 90% ratio of Corin-positive cells on culture day 12 for the induction of differentiation. On the other hand, the cell group A2 had a 41% ratio of Corin-positive cells on culture day 12 for the induction of differentiation. In the analysis of the concentration of NT-3 in the culture supernatant, for the cell group A1, NT-3 was not detected from the culture supernatant samples collected 1 day and 5 days after the start of differentiation and however, specifically increased in the sample collected 3 days thereafter (Fig. 1). On the other hand, for the cell group A2, NT-3 was not detected in the culture supernatant sample collected on any day.

### <Culture experiment B>

The cell group B1 had a 29% ratio of Corin-positive cells on culture day 12 for the induction of differentiation. On the other hand, the cell group B2 had a 65% ratio of Corin-positive cells on culture day 12 for the induction of differentiation. In the analysis of the concentration of NT-3 in the culture supernatant, for the cell group B2, NT-3 was not detected from the culture supernatant samples collected 1 day, 4 days, and 5 days after the start of differentiation and however, specifically increased in the culture supernatant samples collected 2 days and 3 days thereafter (Fig. 2). On the other hand, for the cell group B1, NT-3 was not detected in the culture supernatant sample collected on any day.

### <Culture experiment C>

The cell group C1 had a 24% ratio of Corin-positive cells on culture day 12 for the induction of differentiation. On the other hand, the cell group C2 had an 83% ratio of Corin-positive cells on culture day 12 for the induction of differentiation. As for the concentration of NT-3, for the cell group C2, NT-3 was not detected from the culture supernatant samples collected 1 day and 5 days after the start of differentiation and however, specifically increased in the samples collected 2 days, 3 days, and 4 days thereafter (Fig. 3). On the other hand, for the cell group C1, NT-3 was not detected in the culture supernatant sample collected on any day.

The results of the culture experiments A, B, and C mentioned above suggest that the reference concentration in the first method is preferably set to, for example, 10 pg/ml. The results also suggest that the reference rate of change in the second method is preferably set to, for example, 5 pg/ml·day. The reference concentration is desirably set according to a manufacturing method, a difference such as the type and line of the cells used, and a manufacturing process to be carried out.

### <Culture experiment D>

Culture was performed under the same conditions for culture for the maintenance of an undifferentiated state and conditions for the induction of differentiation as in the culture experiment C. The cell group D1 was subjected to the same culture conditions (deviant conditions) as in the cell group C1, and the cell group D2 was subjected to the same culture conditions (standard conditions) as in the cell group C2.

In the culture experiment D, the cell groups D1 and D2 were subjected to single-cell gene expression analysis in order to analyze the gene expression of NT-3. The cells were collected at the start of differentiation (Day 0) and 4 days, 8 days, and 12 days after the start of differentiation, and single-cell gene expression libraries were prepared. The single-cell gene expression libraries were prepared using Chromium Next GEM Single Cell 3' Reagent Kits v3.1 (10X Genomics, Inc.). Sequencing analysis was conducted using Novaseq6000.

### <Results>

The results of the single-cell gene expression analysis are shown in Fig. 4. The cell group D2 had a 76% ratio of NT-3 gene (NTF3)-positive cells on culture day 4 for the induction of differentiation, whereas the cell group D1 had a 60% ratio of NTF3-positive cells on culture day 4 for the induction of differentiation. Count per Million (CPM) serving as an index for average RNA expression levels was 49 for the cell group D2 and by contrast, was 28 for the cell group D1. As mentioned above, the expression of NT-3 also correlated at the mRNA level with the content of NT-3 in the culture supernatant. It was also evident that the expression of NT-3 is not found only in some subpopulations but temporarily found in a majority of cells in the process of differentiation into cells of interest in association with the induction of differentiation. NT-3 was thus found proper as a monitoring molecule for assessing the induction of differentiation early.

These results demonstrated that the differentiation potential of cells in a culture broth in the differentiation of pluripotent stem cells into neural cells of the midbrain floor plate region can be assessed at an early stage of induction of differentiation by monitoring the concentration of NT-3 secreted from pluripotent stem cells into a culture supernatant, i.e., the concentration of NT-3 secreted into a culture supernatant in the process of the differentiation of pluripotent stem cells into neural cells of the midbrain floor plate region.

<<Experiment 2>>

### «Culture for maintenance of undifferentiated state»

Prior to culture for the induction of differentiation mentioned later, iPS cells were cultured while their undifferentiated state was maintained. Specifically, one or more types of culture conditions of the final stage of an undifferentiated state maintenance step were set under conditions of culture experiments E and F and comparatively studied.

### <Culture experiment E>

An iPS cell line established from human adult peripheral blood mononuclear cells using a Sendai virus vector (CytoTune(TM)-2.0; ID Pharma Co., Ltd.)) was used and cultured under similar differentiation induction conditions as in the cell group A2 in the culture experiment A.

### <Culture experiment F>

An iPS cell line (formal name: 201B7) was used and cultured under similar culture conditions as in the cell group A2 in the culture experiment A. In the culture experiment F, cell groups F1 and F2 differing in medium component were established.

### «Culture for induction of differentiation into neural cells of midbrain floor plate region»

The pluripotent stem cells in each of three types of cell groups provided in the culture experiments E and F were induced to differentiate into neural cells of the midbrain floor plate region in accordance with the same protocol as in Experiment 1 mentioned above.

### <Concentration analysis of NT-3>

A portion of the culture supernatant in each cell group was collected every 24 hours or every 48 hours from immediately after the start of culture for the induction of differentiation (i.e., immediately after the start of differentiation), and analyzed for the concentration of NT-3. In this respect, the culture supernatant obtained in the culture experiment D mentioned above was also analyzed for the concentration of NT-3. The results are shown in Figs. 5 to 7. The concentration of NT-3 in the culture experiment E was analyzed using Bioplex(R) manufactured by Bio-Rad Laboratories, Inc. The concentration of NT-3 in the culture experiments D and F was analyzed using digital ELISA.

### «Results»

### <Culture experiment E>

The cell group used in the culture experiment E had a 44% ratio of Corin-positive cells on culture day 12 for the induction of differentiation. As for the concentration of NT-3, NT-3 was not detected from the culture supernatant samples collected 1 day to 4 days and 9 days to 12 days after the start of differentiation and however, specifically increased in the samples collected 5 days to 8 days thereafter (Fig. 5).

### <Culture experiments D and F>

The cell group D1 had a 34% ratio of Corin-positive cells on culture day 12 for the induction of differentiation. On the other hand, the cell group D2 had a 58% ratio of Corin-positive cells on culture day 12 for the induction of differentiation. As for the concentration of NT-3, for both the cell groups D1 and D2, NT-3 was not detected from the culture supernatant sample collected 1 day after the start of differentiation and however, gradually increased 2 days or later thereafter (Fig. 6). The cell group D2 tended to exhibit a higher concentration of NT-3 than that in the cell group D1 and exhibited correlation with the ratio of Corin-positive cells.

The cell group F1 had a 57% ratio of Corin-positive cells on culture day 12 for the induction of differentiation. On the other hand, the cell group F2 had a 67% ratio of Corin-positive cells on culture day 12 for the induction of differentiation. As for the concentration of NT-3, for both the cell groups F1 and F2, NT-3 was gradually increased 1 day or later after the start of differentiation (Fig. 7). The cell group F2 tended to exhibit a higher concentration of NT-3 than that in the cell group F1 and exhibited correlation with the ratio of Corin-positive cells.

The results of Experiment 2 also demonstrated that the differentiation potential of cells in a culture broth in the differentiation of pluripotent stem cells into neural cells of the midbrain floor plate region can be assessed at an early stage of induction of differentiation by monitoring the concentration of NT-3 secreted from pluripotent stem cells into a culture supernatant.

### «Culture for induction of differentiation into dopaminergic progenitor cells»

Each of six types of cell groups in the culture experiments A, B, and C mentioned above was cultured for the induction of differentiation into dopaminergic progenitor cells by the following method.

Corin-positive cells are collected by flow cytometry analysis using an anti-Corin antibody 12 days after the start of induction of differentiation (Day 12). The collected Corin-positive cells are transferred at 20000 cells/well to Prime Surface 96 U-bottom plate (Sumitomo Bakelite Co., Ltd.) and suspension-cultured using basal medium B (Neurobasal medium (Invitrogen) supplemented with B27 Supplement without vitamin A (Invitrogen), 20 ng/mL BDNF, 10 ng/mL GDNF, 200 mM Ascorbic acid, and 0.4 mM dbcAMP (Sigma)). In this respect, a medium supplemented with 30 µM Y-27632 is used as an initial medium, and a medium supplemented with no Y-27632 is used in replacing half the amount of the medium once three days. The suspension culture is carried out up to 16 days after collection of the Corin-positive cells (Day 28), and FoxA2 and Nurr1 (both are midbrain markers) are stained to confirm differentiation into dopaminergic progenitor cells.

As mentioned above, the embodiments and the examples of the present invention are described. However, it is expected from the beginning that the configurations of each embodiment and each example mentioned above are appropriately combined.

All the embodiments and the examples disclosed herein are given for illustrative purposes and should be considered as non-limiting. The scope of the present invention is shown by claims, not the embodiments and the examples described above, and intends to include the meaning equivalent to that of claims and every change or modification within the scope.

## Claims

1. A method for assessing differentiation potential of cells in a culture broth in the differentiation of pluripotent stem cells into neural cells of the midbrain floor plate region, comprising:
measuring a concentration of NT-3 in a culture supernatant in a culture broth obtained by culturing the pluripotent stem cells in a medium containing an inducer of differentiation into the neural cells of the midbrain floor plate region;
comparing the measured concentration of NT-3 with a reference concentration; and
when the concentration of NT-3 is equal to or more than the reference concentration, assessing the cells in the culture broth as capable of differentiating into the neural cells of the midbrain floor plate region, wherein
the culture supernatant is a culture supernatant collected from the culture broth at any time from 48 hours to 240 hours after the start of culture of the pluripotent stem cells.

2. A method for assessing differentiation potential of cells in a culture broth in the differentiation of pluripotent stem cells into neural cells of the midbrain floor plate region, comprising:
measuring a concentration of NT-3 in each of a first culture supernatant and a second culture supernatant in a culture broth obtained by culturing the pluripotent stem cells in a medium containing an inducer of differentiation into the neural cells of the midbrain floor plate region;
determining a rate of change in the concentration of NT-3 from the concentration of NT-3 in the first culture supernatant and the concentration of NT-3 in the second culture supernatant;
comparing an absolute value of the rate of change in the concentration of NT-3 with a reference rate of change; and
when the absolute value of the rate of change in the concentration of NT-3 is equal to or more than the reference rate of change, assessing the cells in the culture broth as capable of differentiating into the neural cells of the midbrain floor plate region, wherein
the first culture supernatant is a culture supernatant collected from the culture broth at any time from 48 hours to 192 hours after the start of culture of the pluripotent stem cells, and
the second culture supernatant is a culture supernatant collected from the culture broth at any time from 24 hours to 96 hours before collection of the first culture supernatant or at any time from 24 hours to 96 hours after collection of the first culture supernatant.

3. A method for assessing differentiation potential of cells in a culture broth in the differentiation of pluripotent stem cells into neural cells of the midbrain floor plate region, comprising:
measuring a concentration of NT-3 in each of a first culture supernatant, a second culture supernatant, and a third culture supernatant in a culture broth obtained by culturing the pluripotent stem cells in a medium containing an inducer of differentiation into the neural cells of the midbrain floor plate region;
comparing the concentration of NT-3 in the first culture supernatant, the concentration of NT-3 in the second culture supernatant, and the concentration of NT-3 in the third culture supernatant; and
when the concentration of NT-3 in the first culture supernatant is higher than the concentration of NT-3 in the second culture supernatant and the concentration of NT-3 in the third culture supernatant, assessing the cells in the culture broth as capable of differentiating into the neural cells of the midbrain floor plate region, wherein
the first culture supernatant is a culture supernatant collected from the culture broth at any time from 48 hours to 192 hours after the start of culture of the pluripotent stem cells,
the second culture supernatant is a culture supernatant collected from the culture broth at any time from 24 hours to 96 hours before collection of the first culture supernatant, and
the third culture supernatant is a culture supernatant collected from the culture broth at any time from 24 hours to 96 hours after collection of the first culture supernatant.

4. The method for assessing differentiation potential of cells in a culture broth in the differentiation of pluripotent stem cells into neural cells of the midbrain floor plate region according to any one of claims 1 to 3, wherein the inducer of differentiation into the neural cells of the midbrain floor plate region comprises at least one SMAD signaling-inhibiting substance.

5. The method for assessing differentiation potential of cells in a culture broth in the differentiation of pluripotent stem cells into neural cells of the midbrain floor plate region according to claim 4, wherein the SMAD signaling-inhibiting substance comprises at least one BMP signaling-inhibiting substance and at least one TGFβ signaling-inhibiting substance.

6. The method for assessing differentiation potential of cells in a culture broth in the differentiation of pluripotent stem cells into neural cells of the midbrain floor plate region according to claim 5, wherein
the BMP signaling-inhibiting substance comprises at least one member selected from the group consisting of LDN-193189, Noggin, DMH1, Chordin, Follistatin, K02288, LDN-214117, LDN-212854, ML347, and Dorsomorphin, and
the TGFβ signaling-inhibiting substance comprises at least one member selected from the group consisting of SB-431542, A-83-01, Lefty-1, Lefty-2, Galunisertib, SB-202190, SB-525334, SB-505124, NPC30345, Prifenidone, GW788388, E-616452, SD208, TP0427736, BIBF-0775, LY3200882, Vactosertib, ITD-1, SD093, SD908, LY2109761, LY364947, and LY580276.

7. The method for assessing differentiation potential of cells in a culture broth in the differentiation of pluripotent stem cells into neural cells of the midbrain floor plate region according to any one of claims 1 to 6, wherein the inducer of differentiation into the neural cells of the midbrain floor plate region further comprises a SHH signaling-activating substance and/or a Wnt signaling-activating substance.

8. The method for assessing differentiation potential of cells in a culture broth in the differentiation of pluripotent stem cells into neural cells of the midbrain floor plate region according to any one of claims 1 to 6, wherein the medium further contains a SHH signaling-activating substance and a Wnt signaling-activating substance.

9. The method for assessing differentiation potential of cells in a culture broth in the differentiation of pluripotent stem cells into neural cells of the midbrain floor plate region according to claim 7 or 8, wherein the SHH signaling-activating substance comprises at least one member selected from the group consisting of SHH and a fragment and modified form thereof, a SHH receptor, a SHH receptor agonist, Hh-Ag1.5, Smoothened Agonist, 20a-hydroxycholesterol, Purmorphamine, and SAG.

10. The method for assessing differentiation potential of cells in a culture broth in the differentiation of pluripotent stem cells into neural cells of the midbrain floor plate region according to claim 7, 8, or 9, wherein the Wnt signaling-activating substance comprises at least one member selected from the group consisting of WNT3A, and a GSK-3β-inhibiting substance.

11. The method for assessing differentiation potential of cells in a culture broth in the differentiation of pluripotent stem cells into neural cells of the midbrain floor plate region according to claim 10, wherein the GSK-3β-inhibiting substance comprises at least one member selected from the group consisting of CHIR99021, BIO, CHIR98014, SKL2001, SB216763, GSK-3β inhibitor VII, and L803-mts.

12. The method for assessing differentiation potential of cells in a culture broth in the differentiation of pluripotent stem cells into neural cells of the midbrain floor plate region according to any one of claims 1 to 11, wherein the pluripotent stem cells are iPS cells or ES cells.

13. A method for manufacturing dopaminergic progenitor cells or precursor cells thereof from pluripotent stem cells, comprising:
(A) starting the culture of the pluripotent stem cells under culture conditions capable of inducing differentiation into neural cells of the midbrain floor plate region;
(B) performing a method for assessing differentiation potential of cells in a culture broth in the differentiation of pluripotent stem cells into neural cells of the midbrain floor plate region according to any one of claims 1 to 12 between 0 hours and 288 hours after the start of culture of the pluripotent stem cells;
(C) determining to further continue the culture of the cells in the culture broth assessed as capable of differentiating into the neural cells of the midbrain floor plate region in the step (B), and allowing the cells in the culture broth to differentiate into the neural cells of the midbrain floor plate region; and
(D) culturing the neural cells of the midbrain floor plate region under culture conditions capable of inducing differentiation into the dopaminergic progenitor cells so that the cells are allowed to differentiate into the dopaminergic progenitor cells or precursor cells thereof.

14. The method for manufacturing dopaminergic progenitor cells or precursor cells thereof from pluripotent stem cells according to claim 13, further comprising, between the step (C) and the step (D),
(E) collecting the neural cells of the midbrain floor plate region obtained in the step (C).

15. The method for manufacturing dopaminergic progenitor cells or precursor cells thereof from pluripotent stem cells according to claim 13 or 14, wherein the step (D) is performed by selecting and collecting midbrain floor plate cells from the neural cells of the midbrain floor plate region, and culturing the collected midbrain floor plate cells under culture conditions capable of inducing differentiation into the dopaminergic progenitor cells.
